# EUROPÄISCHE PATENTSCHRIFT

(11) **EP 1 218 430 B1**
(45) Veröffentlichungstag und Bekanntmachung des Hinweises auf die Patenterteilung: **29.03.2006**
(21) Anmeldenummer: 00964064.0
(22) Anmeldetag: 30.08.2000
(51) Int. Cl.: C08G 18/08, C08G 18/61, A61K 8/87

(54) **KOSMETISCHES MITTEL AUF BASIS VON URETHAN- UND/ODER HARNSTOFFGRUPPEN-HALTIGEN OLIGOMEREN**
COSMETIC BASED ON OLIGOMERS CONTAINING URETHANE AND/OR UREA FUNCTIONAL GROUP
AGENTS COSMETIQUES SE BASANT SUR DES OLIGOMERES COMPORTANT DES GROUPES URETHANE ET/OU UREE

(30) Priorität: 31.08.1999 DE 19941365
(43) Veröffentlichungstag der Anmeldung: 03.07.2002
(73) Patentinhaber: BASF Aktiengesellschaft, 67056 Ludwigshafen (DE)
(72) Erfinder: NGUYEN KIM, Son, 69502 Hemsbach (DE); SANNER, Axel, 67227 Frankenthal (DE); HÖSSEL, Peter, 67105 Schifferstadt (DE); MEYER, Hans, Joachim, 67229 Gerolsheim (DE)
(74) Vertreter: Kinzebach, Werner
(86) Internationale Anmeldenummer: PCT/EP2000/008456
(87) Internationale Veröffentlichungsnummer: WO 2001/016200

(56) Entgegenhaltungen:
- EP-A- 0 619 111
- EP-A- 0 751 162
- WO-A-98/11854

## Beschreibung

Die vorliegende Erfindung betrifft Oligomere und Polymere mit Urethan- und/oder Harnstoffgruppen, die Verwendung dieser Oligomere in pharmazeutischen und kosmetischen Zubereitungen und kosmetische Mittel, die diese Oligomere und Polymere enthalten.

In der Kosmetik werden Polymere mit filmbildenden Eigenschaften zur Festigung, Strukturverbesserung und Formgebung der Haare verwendet. Diese Haarbehandlungsmittel enthalten im Allgemeinen eine Lösung des Filmbildners in einem Alkohol oder einem Gemisch aus Alkohol und Wasser.

Haarfestigungsmittel werden im Allgemeinen in Form von wässrig-alkoholischen Lösungen auf die Haare aufgesprüht. Nach dem Verdampfen des Lösungsmittels werden die Haare an den gegenseitigen Berührungspunkten vom zurückbleibenden Polymer in der gewünschten Form gehalten. Die Polymere sollen einerseits so hydrophil sein, dass sie aus dem Haar ausgewaschen werden können, andererseits aber sollen sie hydrophob sein, damit die mit den Polymeren behandelten Haare auch bei hoher Luftfeuchtigkeit ihre Form behalten und nicht miteinander verkleben. Um eine möglichst effiziente Haarfestigerwirkung zu erzielen, ist es außerdem wünschenswert, Polymere einzusetzen, welche ein relativ hohes Molekulargewicht und eine relativ hohe Glastemperatur (mindestens 10 °C) besitzen.

Ein weiterer aktueller Anspruch an Haarbehandlungsmittel ist es, dem Haar Flexibilität, ein natürliches Aussehen und Glanz zu verleihen, z. B. auch dann, wenn es sich um von Natur aus besonders kräftiges und/oder dunkles Haar handelt.

Bei der Formulierung von Haarfestigern ist außerdem zu berücksichtigen, dass aufgrund der Umweltbestimmungen zur Kontrolle der Emission flüchtiger organischer Verbindungen (VOC = volatile organic compounds) in die Atmosphäre eine Verringerung des Alkohol- und Treibmittelgehalts erforderlich ist.

Die DE-A-42 25 045 und die WO 94/03515 beschreiben die Verwendung von wasserlöslichen oder in Wasser dispergierbaren, anionischen Polyurethanen als Haarfestiger. Diese Polyurethane sind aufgebaut aus
a) mindestens einer Verbindung, die zwei oder mehrere aktive Wasserstoffatome pro Molekül enthält,
b) mindestens einem Säure- oder Salzgruppen enthaltenden Diol und
c) mindestens einem Diisocyanat.

Die in diesen Polyurethanen enthaltenen Säuregruppen können durch Neutralisation mit wenigstens einer Base in die entsprechenden Salze überführt werden. Dazu werden niedermolekulare Amine, wie 2-Amino-2-methylpropanol, Diethylaminopropylamin und Trüsopropanolamin eingesetzt.

Die EP-A-619 111 beschreibt die Verwendung von Polyurethanen auf Basis von organischen Diisocyanaten, Diolen und 2,2-Hydroxymethyl-substituierten Carboxylaten in Haarfixierungsmitteln. Zumindest ein Teil der Carbonsäuregruppen wird dabei mit einer organischen oder anorganischen Base, ausgewählt unter Natriumhydroxid, Kaliumhydroxid, 2-Amino-2-methylpropanol, Histidin, Tris(hydroxymethyl)aminomethan und Triethanolamin, neutralisiert.

Die DE-A-195 41 658 beschreibt wasserlösliche bzw. wasserdispergierbare Pfropfpolymere aus einem Polyurethanpräpolymer mit endständigen Isocyanatgruppen und einem freie Aminogruppen enthaltenden Protein.

Die EP-A-636 361 beschreibt eine kosmetische Zusammensetzung, welche in einem kosmetisch verträglichen Träger mindestens einen Pseudolatex auf Basis eines Polykondensates umfasst, das mindestens eine Polysiloxaneinheit und mindestens eine Polyurethan- und/oder Polyharnstoffeinheit mit anionischen oder kationischen Gruppen umfasst. Als Neutralisierungsmittel werden dabei Mineralbasen, niedermolekulare Amine und Aminoalkohole, Mineralsäuren und niedermolekulare Carbonsäuren eingesetzt. Die WO 97/25021 hat einen vergleichbaren Offenbarungsgehalt. Die Auswaschbarkeit dieser Filmbildner ist nicht zufriedenstellend. Zudem besitzen sie aufgrund eines hohen Siloxananteils auch nicht die für ein Haarpolymer erforderliche Festigungswirkung.

Die DE-A-195 41 329 und die WO 97/17052 beschreiben Haarbehandlungsmittel, enthaltend ein in Wasser oder in einem Wasser/Alkohol-Gemisch lösliches oder dispergierbares Haarfestigerpolymer und zusätzlich ein in Wasser lösliches oder dispergierbares siloxanhaltiges Salz. Haarspray-Formulierungen auf Basis dieser siloxanhaltigen Salze, einem nicht siloxanhaltigen Haarfestigerpolymer und einem Siliconöl führen zu Filmen, die leicht, z. B. durch mechanische Belastung, von der Haaroberfläche abgelöst werden. Die Festigungswirkung dieser Formulierungen ist daher verbesserungswürdig.

Die DE-A-195 41 326 und die WO 97/17386 beschreiben wasserlösliche oder wasserdispergierbare Polyurethane mit endständigen Säuregruppen, ihre Herstellung und ihre Verwendung. Dabei wird ein in Wasser lösliches oder dispergierbares Polyurethanpräpolymer mit endständigen Isocyanatgruppen mit einer Aminosulfonsäure oder Aminocarbonsäure, insbesondere Taurin, Asparaginsäure und Glutaminsäure, umgesetzt.

Keines der zuvor genannten Dokumente beschreibt Polyurethane auf Basis von Diisocyanaten und Oligomeren mit gegenüber Isocyanatgruppen reaktiven Gruppen, wobei diese Oligomere ihrerseits Urethan- und/oder Harnstoffgruppen eingebaut enthalten. Auch der Einsatz solcher Oligomere in polymergebundener und nicht-polymergebundener Form in kosmetischen Produkten wird nicht beschrieben.

An kosmetische und pharmazeutische Mittel zur Behandlung von Haut und Haaren werden spezielle Anforderungen bezüglich ihrer rheologischen Eigenschaften gestellt. So ist es z. B. ein aktueller Anspruch an Hautpflegeprodukte, einen hohen Anteil an pflegenden Substanzen aufzuweisen. Dabei handelt es sich oftmals um Verbindungen, wie natürliche Öle und Fette, etherische Öle etc., die aufgrund ihrer Fließeigenschaften nur mit Hilfe von Zusatzstoffen in die gewünschte Anwendungsform, wie z. B. Stiftform, gebracht werden können. Auch an Haarpflegeprodukte, z. B. Shampoos, werden spezielle Ansprüche bezüglich ihrer Viskosität gestellt. Im Allgemeinen sollen solche Produkte eine höhere Viskosität bzw. ein festere Konsistenz aufweisen, als dies mit den eingesetzten Wirk- und Pflegesubstanzen alleine erzielt wird. Vorzugsweise sollen Zusatzstoffe zur Einstellung der Viskosität bzw. der Konsistenz in möglichst geringen Mengen eingesetzt werden.

Die WO-A 98/17705 beschreibt eine Harzzusammensetzung, die esterterminierte Polyamide enthält und deren Verwendung zur Formulierung transparenter Gele aus flüssigen Kohlenwasserstoffen mit geringer Polarität.

Die WO-A 97/36572 beschreibt eine Basiszusammensetzung zur Herstellung kosmetischer Mittel, die wenigstens ein flüssiges Silicon und wenigstens ein gelbildendes Mittel enthält. Das flüssige Silicon wird dabei in Mengen von 0,5 bis 95 Gew.-%, bezogen auf das Gesamtgewicht der Zusammensetzung eingesetzt, die mit Ausführungsbeispielen belegten Formulierungen enthalten mindestens 27 Gew.-%. Als Gelbildner werden Polymere eingesetzt, die zwingend Siloxangruppen und polare, zur Ausbildung von Wasserstoffbrückenbindungen geeignete Gruppen eingebaut enthalten. Letztere sind ausgewählt unter Estergruppen, Urethangruppen, Harnstoffgruppen, Thioharnstoffgruppen und Amidgruppen. Die als Gelbildner eingesetzten Polymere eignen sich dabei nicht als Verdicker für Öle, die nicht auf Silicon basieren. Auch die Verdickung von Ölgemischen aus Siliconölen und davon verschiedenen weiteren Ölen wird in dieser Schrift nicht beschrieben.

Die WO 98/11854 beschreibt hydrophile Polyetherurethane, die durch Reaktion eines langkettigen Polyalkylendiols, eines Glycols, eines organischen Diisocyanats, einer 2,2-Di-(hydroxymethyl)alkancarbonsäure und einer geringen Menge Wasser erhältlich sind.

Der vorliegenden Erfindung liegt die Aufgabe zugrunde, neue kosmetische Mittel, insbesondere zur Behandlung von Haut und Haaren, zur Verfügung zu stellen. Bevorzugt sollen die kosmetischen Mittel gute rheologische Eigenschaften aufweisen. Kosmetische Mittel in Form von Haarbehandlungsmitteln sollen klebfreie Filme mit guten flexiblen Eigenschaften bilden. Vorzugsweise sollen diese Mittel dem Haar Glätte und Geschmeidigkeit verleihen.

Überraschenderweise wurde nun gefunden, dass diese Aufgabe durch kosmetische Mittel gelöst wird, die wenigstens ein Oligomer enthalten, das mindestens ein Diisocyanat und mindestens eine Verbindung mit wenigstens zwei gegenüber Isocyanatgruppen reaktiven Gruppen eingebaut enthält, wobei das Oligomer pro Molekül wenigstens zwei Urethan- und/oder Harnstoffgruppen und zusätzlich wenigstens zwei weitere funktionelle Gruppen mit aktiven Wasserstoffatomen umfasst.

Gegenstand der vorliegenden Erfindung ist daher ein kosmetisches Mittel, enthaltend:
wenigstens ein Oligomer aus
A) mindestens einem aliphatischen Diisocyanat,
B) mindestens einer Verbindung mit wenigstens zwei gegenüber Isocyanatgruppen reaktiven Gruppen, die ausgewählt ist unter
   B1) aliphatischen und cycloaliphatischen Polyolen, Polyaminen und/oder Aminoalkoholen,
   B2) Polyetherolen und/oder Diaminopolyethern,
   B3) Polysiloxanen mit wenigstens zwei aktiven Wasser-stoffatomen pro Molekül,
   B4) Polyesterpolyolen,
   und Mischungen davon, und
C) gegebenenfalls mindestens einer Dicarbonsäure und/oder Hydroxycarbonsäure,
wobei das Oligomer pro Molekül wenigstens zwei Urethan- und/oder Harnstoffgruppen und zusätzlich wenigstens zwei weitere funktionelle Gruppen, die ausgewählt sind unter Hydroxyl-, primären und/oder sekundären Aminogruppen, umfasst und wobei Oligomere aus den Komponenten A) und B) ein Molekulargewicht im Bereich von 500 bis 7000 und Oligomere aus den Komponenten A), B) und C) ein Molekulargewicht im Bereich von 500 bis 10000 aufweisen.

Nach einer ersten bevorzugten Ausführungsform enthalten die erfindungsgemäßen kosmetischen Mittel wenigstens ein Oligomer wie zuvor beschrieben.

Die erfindungsgemäßen kosmetischen Mittel enthalten die Oligomere vorzugsweise in Form einer separaten Komponente und/oder eingebaut in ein Polymer. Bevorzugte Polymere, die die Oligomere in eingebauter Form enthalten, sind Polyurethane. Im Rahmen der vorliegenden Erfindung umfasst der Ausdruck "Polyurethane" auch Polymere, die Harnstoffgruppen statt oder zusätzlich zu den Urethangruppen aufweisen.

Bevorzugt weist das Oligomer 2 bis 50, insbesondere 3 bis 45 Urethan- und/oder Harnstoffgruppen pro Molekül auf.

Erfindungsgemäß werden Oligomere aus den Komponenten A) und B) eingesetzt, die ein Molekulargewicht im Bereich von 500, bevorzugt 600, insbesondere 700, bis 7000 ausfweisen.

Weiterhin werden Oligomere aus den Komponenten A), B) und C) eingesetzt, die ein Molekulargewicht im Bereich von 500, bevorzugt 600 insbesondere 700 bis 10000 aufweisen.

Hydroxylgruppenhaltige Oligomere weisen vorzugsweise eine Alkoholzahl (OH-Zahl) von etwa 5 bis 150 mg KOH/g, besonders bevorzugt 10 bis 150 mg KOH/g, insbesondere 20 bis 100 mg KOH/g, auf. Amingruppenhaltige Oligomere weisen vorzugsweise eine Aminzahl von etwa 5 bis 150 mg KOH/g, besonders bevorzugt 10 bis 150 mg KOH/g, besonders bevorzugt 20 bis 100 mg KOH/g, auf. Bei Oligomeren, die sowohl Hydroxyl- als auch Aminogruppen aufweisen, liegt die Summe aus Alkohol- und Aminzahl vorzugsweise in einem Bereich von etwa 5 bis 150, besonders bevorzugt 10 bis 150, insbesondere 20 bis 100.

Bevorzugt weisen die Oligomere keine freien Isocyanatgruppen auf.

Vorteilhafterweise zeigen die erfindungsgemäßen kosmetischen Mittel auf Basis der genannten Oligomere im Allgemeinen auch ohne den Zusatz von silicongruppenhaltigen Komponenten gute anwendungstechnische Eigenschaften. So verleihen z. B. Haarbehandlungsmittel, bei denen das Oligomer und/oder die übrigen Komponenten der Formulierung siliconfrei sind, dem Haar im Allgemeinen dennoch eine gute Flexibilität. Nach einer bevorzugten Ausführungsform enthalten die erfindungsgemäßen kosmetischen Mittel wenigstens ein Oligomer, das keine Silicongruppen umfasst. Nach einer weiteren bevorzugten Ausführungsform enthalten die erfindungsgemäßen kosmetischen Mittel ein silicongruppenhaltiges Oligomer, wobei die übrigen Komponenten des Mittels siliconfrei sind. Insbesondere sind sowohl das Oligomer als auch die übrigen Komponenten des kosmetischen Mittels siliconfrei. Eine weitere geeignete Ausführungsform sind kosmetische Mittel, bei denen das Oligomer und/oder wenigstens eine weitere Komponente Silicongruppen enthalten.

Die Diisocyanate A) sind ausgewählt unter aliphatischen Diisocyanaten, wie Tetramethylendiisocyanat und Hexamethylendiisocyanat. Besonders bevorzugt wird Hexamethylendiisocyanat eingesetzt. Gewünschtenfalls können bis zu 3 Mol-% der genannten Verbindungen durch Triisocyanate ersetzt sein.

Vorzugsweise handelt es sich bei den gegenüber Isocyanatgruppen reaktiven Gruppen der Komponente B) um Hydroxylgruppen, primäre und/oder sekundäre Aminogruppen.

Bevorzugt werden als Polyolkomponente B1) Diole eingesetzt, deren Molekulargewicht in einem Bereich von etwa 62 bis 500 g/mol liegt. Dazu zählen z. B. Diole mit 2 bis 18 Kohlenstoffatomen, vorzugsweise 2 bis 10 Kohlenstoffatomen, wie 1,2-Ethandiol, 1,3-Propandiol, 1,4-Butandiol, 1,6-Hexandiol, 1,5-Pentandiol, 1,10-Decandiol, 2-Methyl-1,3-propandiol, 2,2-Dimethyl-1,3-propandiol, Di-, Tri-, Tetra-, Penta- und Hexaethylenglykol, Neopentylglykol, Cyclohexandimethylol, Glycerinmonostearat und Mischungen davon.

Bevorzugt werden als Komponente B1) weiterhin Triole und höherwertige Polyole mit 3 bis 100, bevorzugt 3 bis 70 Kohlenstoffatomen, eingesetzt. Bevorzugte Triole sind z. B. Glycerin und Trimethylolpropan. Bevorzugte Triole B1) sind weiterhin die Triester von Hydroxycarbonsäuren mit dreiwertigen Alkoholen. Vorzugsweise handelt es sich dabei um Triglyceride von Hydroxycarbonsäuren, wie z. B. Milchsäure, Hydroxystearinsäure und Ricinolsäure. Geeignet sind auch natürlich vorkommende Gemische, die Hydroxycarbonsäuretriglyceride enthalten, insbesondere Ricinusöl. Bevorzugte höherwertige Polyole B1) sind z. B. Erythrit, Pentaerythrit und Sorbit.

Bevorzugte Aminoalkohole B1) sind z. B. 2-Aminoethanol, 2-(N-Methylamino)ethanol, 3-Aminopropanol, 4-Aminobutanol, 1-Ethylaminobutan-2-ol, 2-Amino-2-methyl-1-propanol, 4-Methyl-4-aminopentan-2-ol etc.

Bevorzugte Polyamine B1) sind z. B. Diamine, wie Ethylendiamin, Propylendiamin, 1,4-Diaminobutan, 1,5-Diaminopentan und 1,6-Diaminohexan. Bevorzugte Triamine B1) sind z. B. Di-ethylentriamin, N,N'-Diethyldiethylentriamin etc. Bevorzugte höherwertige Polyamine sind z. B. Triethylentetramin etc.

Die als Komponente B1) genannten Verbindungen können einzeln oder in Mischungen eingesetzt werden. Besonders bevorzugt werden 1,2-Ethandiol, 1,4-Butandiol, 1,6-Hexandiol, Neopentylglykol, Diethylenglykol, Cyclohexandimethylol und Mischungen davon eingesetzt.

Bei der Komponente B2) handelt es sich bevorzugt um ein Polyetherol mit einem zahlenmittleren Molekulargewicht im Bereich von etwa 300 bis 5 000, bevorzugt etwa 400 bis 4000, insbesondere 500 bis 1500. Bevorzugte Polyetherole sind Polyalkylenglykole, z. B. Polyethylenglykole, Polypropylenglykole, Polytetrahydrofurane, Copolymerisate aus Ethylenoxid, Propylenoxid und/oder Butylenoxid, die die Alkylenoxideinheiten statistisch verteilt oder in Form von Blöcken einpolymerisiert enthalten, etc. Vorzugsweise werden als Komponente B2) Polytetrahydrofurane und Mischungen, die diese enthalten, eingesetzt.

Geeignete Polytetrahydrofurane B2) können durch kationische Polymerisation von Tetrahydrofuran in Gegenwart von sauren Katalysatoren, wie z. B. Schwefelsäure oder Fluoroschwefelsäure, hergestellt werden. Derartige Herstellungsverfahren sind dem Fachmann bekannt.

Bei den Polysiloxanen B3) handelt es sich vorzugsweise um eine Verbindung der Formel II 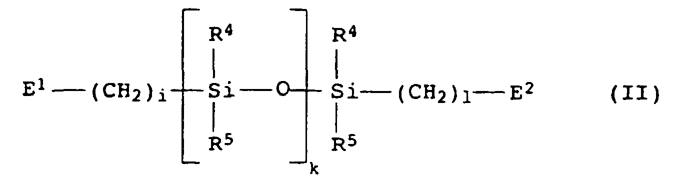 worin
- R⁴ und R⁵: unabhängig voneinander für C₁- bis C₄-Alkyl, Benzyl oder Phenyl stehen,
- E¹ und E²: unabhängig voneinander für OH oder NHR⁶ stehen, wobei R⁶ für Wasserstoff, C₁- bis C₆-Alkyl oder C₅- bis C₈-Cycloalkyl steht,
- i und l: unabhängig voneinander für 2 bis 8 stehen,
- k: für 3 bis 50 steht,
und Mischungen davon.

Geeignete Alkylreste sind z. B. Methyl, Ethyl, n-Propyl, Isopropyl, n-Butyl, t.-Butyl, n-Pentyl oder n-Hexyl. Geeignete Cycloalkylreste sind z. B. Cyclopentyl, Cyclohexyl, Cycloheptyl oder Cyclooctyl.

Vorzugsweise stehen R⁴ und R⁵ beide für Methyl.

Diese Polysiloxane B3) weisen vorzugsweise ein zahlenmittleres Molekulargewicht im Bereich von etwa 300 bis 5000, bevorzugt 400 bis 3000, auf.

Bei den Polysiloxanen B3) handelt es sich weiterhin vorzugsweise um eine Verbindung der Formel III 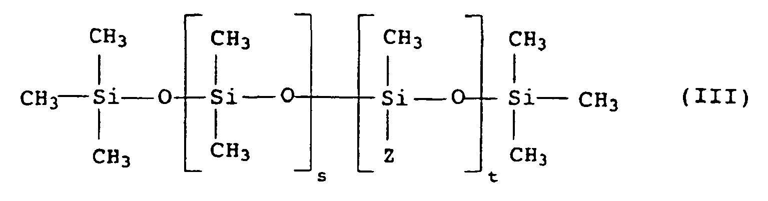 worin
die Reihenfolge der Siloxaneinheiten beliebig ist,
- s: für einen Wert von 5 bis 200, bevorzugt 10 bis 100, steht,
- t: für einen Wert von 1 bis 20, bevorzugt 2 bis 10, steht,
- Z: für einen Rest der Formel -(CH₂)ᵤ-NH₂ steht, worin u für eine ganze Zahl von 1 bis 10, bevorzugt 2 bis 6 steht, oder
Z für einen Rest der Formel -(CH₂)ₓ-NH-(CH₂)_{y}-NH₂ steht, worin x und y unabhängig voneinander für 0 bis 10, bevorzugt 1 bis 6, stehen, wobei die Summe aus x und y für 1 bis 10, bevorzugt 2 bis 6, steht.

Dazu zählen z. B. die MAN- und MAR-Marken der Fa. Hüls sowie die Finish-Marken der Fa. wacker, z. B. Finish WT 1270.

Geeignete Verbindungen B3) sind auch die in der EP-A-227 816 beschriebenen Polydimethylsiloxane, auf die hiermit Bezug genommen wird.

Geeignete Polyesterpolyole B4) sind lineare und verzweigte Polymere mit endständigen OH-Gruppen, z. B. solche mit mindestens zwei OH-Gruppen. Polyesterpolyole lassen sich z. B. durch Veresterung von aliphatischen, cycloaliphatischen und aromatischen Di-, Tri- und/oder Polycarbonsäuren sowie von Hydroxycarbonsäuren mit Di-, Tri- und/oder Polyolen herstellen. Bevorzugte Polyesterole B4) sind Polyesterdiole.

Vorzugsweise weisen die Polyesterole B4) ein zahlenmittleres Molekulargewicht im Bereich von etwa 400 bis 5000, bevorzugt 500 bis 4000, insbesondere 600 bis 3000, auf.

Bevorzugt ist die Komponente B4) ausgewählt unter Estern zwei- oder mehrwertiger Alkohole mit mindestens einer Carbonsäure, wobei die Carbonsäure ausgewählt ist unter
- cyclischen oder acyclischen Dicarbonsäuren, erhalten durch Dimerisierung ungesättigter C₆- bis C₃₀-Carbonsäuren,
- aliphatischen, cycloaliphatischen und/oder aromatischen C₈- bis C₃₀-Dicarbonsäuren,
- aliphatischen, cycloaliphatischen und aromatischen C₈- bis C₃₀-Hydroxycarbonsäuren,
und Mischungen davon.

Durch Dimerisierung ein- oder mehrfach ungesättigter Carbonsäuren werden Gemische aus acyclischen und cyclischen Dicarbonsäuren erhalten, die als dimere Säuren bzw. Dimerfettsäuren bezeichnet werden. Bevorzugt werden zur Herstellung der Komponente B4) Dimerfettsäuregemische eingesetzt, die Dimerfettsäuren mit 8 bis 54 Kohlenstoffatomen enthalten. Bevorzugt sind Dimerfettsäuren mit durchschnittlich 36 Kohlenstoffatomen, die z. B. bei der Dimerisierung von ungesättigten C₁₈-Fettsäuren enthalten werden. Dimerfettsäuren sind kommerziell erhältlich und erhalten produktionsbedingt Anteile von verzweigten Monofettsäuren und Trimerfettsäuren. Diese können gewünschtenfalls vor dem Einsatz der Dimersäuren zur Herstellung der Komponente B4) destillativ abgetrennt werden. Zur Herstellung der Komponente B4) werden die Dimerfettsäuren mit Diolen, Triolen und/oder Polyolen verestert. Vorzugsweise werden zur Herstellung der Komponente B4) Dimerfettsäuren mit aliphatischen und/oder cycloaliphatischen Diolen, Triolen und/oder Polyolen verestert, wie sie zuvor als Komponente B1) beschrieben sind. Gewünschtenfalls können die Alkohole dabei allein oder in Mischungen eingesetzt werden.

Bevorzugt als Komponente B4) sind weiterhin Polyesterdiole, insbesondere solche auf Basis aromatischer Dicarbonsäuren, wie Terephthalsäure, Isophthalsäure, Phthalsäure, Na- oder K-Sulfoisophthalsäur , aliphatischer Dicarbonsäuren, wie Adipinsäure oder Bernsteinsäure., und cycloaliphatischer Dicarbonsäuren, wie 1,2-, 1,3- oder 1,4-Cyclohexandicarbonsäure. Als Diolkomponente dieser Polyesterdiole B4) kommen insbesondere aliphatische Diole in Betracht, wie Ethylenglykol, Propylenglykol, 1,6-Hexandiol, Neopentylglykol, Diethylenglykol, Polyethylenglykole, Polypropylenglykole, 1,4-Dimethylolcyclohexan.

Bevorzugt sind Polyesterdiole B4) auf Basis von aromatischen und aliphatischen Dicarbonsäuren und aliphatischen Diolen, insbesondere solche, bei denen die aromatische Dicarbonsäure 10 bis 95 Mol-%, insbesondere 40 bis 90 Mol-% des gesamten Dicarbonsäureanteils (Rest aliphatische Dicarbonsäuren) ausmacht.

Besonders bevorzugte Polyesterdiole B4) sind die Umsetzungsprodukte aus Phthalsäure/Diethylenglykol, Isophthalsäure/1,4-Butandiol, Isophthalsäure/Adipinsäure/1,6-Hexandiol, 5-NaSO₃-Isophthalsäure/Phthalsäure/Adipinsäure/1,6-Hexandiol, Adipinsäure/Ethylenglykol, Isophthalsäure/Adipinsäure/Neopentylglykol, Isophthalsäure/Adipinsäure/Neopentylglykol/Diethylenglykol/Dimethylolcyclohexan und 5-NaSO₃-Isophthalsäure/Isophthalsäure/Adipinsäure/Neopentylglykol/Diethylenglykol/Dimethylolcyclohexan, Isophthalsäure/Adipinsäure, Neopentylglykol/Dimethylolcyclohexan.

Bevorzugt als Komponente B4) sind weiterhin Polyesterdiole auf Basis von linearen oder verzweigten, C₈- bis C₃₀-Di- oder Polycarbonsäuren. Diese können gewünschtenfalls eine oder mehrere zusätzliche funktionelle Gruppen, wie z. B. Hydroxylgruppen, aufweisen. Geeignete Di- und Polycarbonsäuren sind z. B. Acelainsäure, Dodecandisäure, Korksäure, Pimelinsäure, Sebacinsäure, Tetradecandisäure, Citronensäure, Ricinolsäure, Hydroxystearinsäure und Gemische davon. Bevorzugte Diole zur Herstellung dieser Komponente B4) sind z. B. 1,6-Hexandiol, Neopentylglykol, 1,4-Dimethylolcyclohexan, Diethylenglykol, Glycerinmonostearat und Gemische davon.

Nach einer bevorzugten Ausführungsform enthalten die erfindungsgemäßen kosmetischen Mittel wenigstens ein Oligomer aus dem Komponenten A) und B), welches mindestens zwei endständige Gruppen mit aktiven Wasserstoffatomen aufweist, die ausgewählt sind unter Hydroxyl-, primären und/oder sekundären Aminogruppen.

Vorzugsweise enthalten die erfindungsgemäßen kosmetischen Mittel wenigstens ein Oligomer, welches mindestens eine Komponente B4) eingebaut enthält, die ausgewählt ist unter Estern zwei- oder mehrwertiger Alkohole mit mindestens einer Carbonsäure, wobei die Carbonsäure ausgewählt ist unter
- cyclischen und acyclischen Dicarbonsäuren, erhalten durch Dimerisierung ungesättigter C₆- bis C₃₀-Carbonsäuren,
- aliphatischen, cycloaliphatischen und aromatischen C₈- bis C₃₀-Dicarbonsäuren,
- C₈- bis C₃₀-Hydroxycarbonsäuren
und Mischungen davon.

Nach einer bevorzugten Ausführungsform enthalten die erfindungsgemäßen kosmetischen Mittel wenigstens ein Oligomer aus den Komponenten A) und B4).

Nach einer weiteren bevorzugten Ausführungsform enthalten die erfindungsgemäßen kosmetischen Mittel wenigstens ein Oligomer aus den Komponenten A), B) und C). Bevorzugt ist die Carbonsäure C) ausgewählt unter den zuvor genannten cyclischen und acyclischen Dicarbonsäuren, erhalten durch Dimerisierung ungesättigter C₆- bis C₃₀-Carbonsäuren, aliphatischen, cycloaliphatischen und aromatischen C₈- bis C₃₀-Dicarbonsäuren, C₈- bis C₃₀-Hydroxycarbonsäuren und Mischungen davon. Besonders bevorzugt ist die Komponente C) ausgewählt unter Sebacinsäure, Acelainsäure, C₃₆-Dimerfettsäure, Ricinolsäure, Hydroxystearinsäure und Gemischen davon.

Die Oligomere auf Basis mindestens einer Carbonsäure C) weisen in Abhängigkeit von den funktionellen Gruppen der Komponente B) Carbonsäureester- und/oder Carbonsäureamidgruppen auf.

Vorzugsweise enthalten die kosmetischen Mittel wenigstens ein Oligomer aus den Komponenten A), B1) und C).

Bevorzugt sind Oligomere aus den Komponenten A), B1) und C), die erhältlich sind durch Umsetzung mindestens einer Verbindung der allgemeinen Formel I

HO-R¹-A¹-CO-MH-R²-MH-CO-A¹-R¹-OH (I)

worin
- R¹: für C₂- bis C₁₂-Alkylen, C₅- bis C₈-Cycloalkylen oder Arylen steht, wobei Alkylenreste durch einen oder zwei C₅- bis C₈-Cycloalkylen- oder Arylenreste unterbrochen sein können,
- R²: für einen von einem aliphatischen, cycloaliphatischen oder aromatischen Diisocyanat nach Entfernen der Isocyanatgruppen abgeleiteten Rest steht,
- A¹: für O oder NR³ steht, wobei R³ für Wasserstoff, C₁- bis C₆-Alkyl oder C₅- bis C₈-Cycloalkyl steht,
mit mindestens einer Carbonsäure C), die ausgewählt ist unter cyclischen oder acyclischen Dicarbonsäuren, erhalten durch Dimerisierung ungesättigter C₆- bis C₃₀-Carbonsäuren, aliphatischen, cycloaliphatischen und aromatischen C₈- bis C₃₀-Dicarbonsäuren, C₈- bis C₃₀-Hydroxycarbonsäuren, und Mischungen davon. In der Formel I kann R¹ auch für einen von Glycerinmonostearat durch Entfernen der OH-Gruppen abgeleiteten Rest stehen.

Zur Herstellung dieser Oligomeren der Formel I wird vorzugsweise wenigstens ein Diisocyanat A) der Formel OCN-R²-NCO mit wenigstens einer Verbindung B1) der Formel HO-R¹-A¹-H umgesetzt. Dabei besitzen die Reste R¹, R² und A¹ die zuvor angegebenen Bedeutungen.

vorzugsweise werden zur Herstellung der Verbindungen der allgemeinen Formel I Diisocyanate A) eingesetzt, die ausgewählt sind unter aliphatischen Diisocyanaten, wie Tetramethylendiisocyanat, Hexamethylendiisocyanat und Gemischen, die diese enthalten.

Vorzugsweise werden zur Herstellung der Verbindungen der allgemeinen Formel I Diole und Aminoalkohole der Formel HO-R¹-A¹-H eingesetzt, die ausgewählt sind unter den zuvor als Komponente B1) genannten Diolen und Aminoalkoholen. Besonders bevorzugt sind aliphatische Diole und Aminoalkohole, wie 1,4-Butandiol, 1,6-Hexandiol, 4-Aminobutanol, 6-Aminohexanol und Gemische davon.

Vorzugsweise liegt das Molmengenverhältnis von Diisocyanat zu Verbindung der Formel HO-R¹-A¹-H in einem Bereich von 1:1,1 bis 1:2,5, bevorzugt bei etwa 1:2.

Die Herstellung der in den erfindungsgemäßen kosmetischen Mitteln enthaltenen Oligomere aus den Komponenten A) und B) erfolgt durch Umsetzung wenigstens eines Diisocyanates A) mit den gegenüber Isocyanatgruppen reaktiven Gruppen der Komponente B). Werden zur Umsetzung Hydroxylgruppen-haltige Komponenten B) eingesetzt, so erfolgt die Umsetzung im Allgemeinen bei einer erhöhten Temperatur im Bereich von etwa 40 bis 150 °C, bevorzugt etwa 70 bis 120 °C. Die Reaktion kann ohne Lösungsmittel in der Schmelze oder in einem geeigneten Lösungsmittel oder Lösungsmittelgemisch erfolgen. Geeignete Lösungsmittel sind aprotisch polare Lösungsmittel, z. B. Tetrahydrofuran, Essigsäureethylester, N-Methylpyrrolidon, Dimethylformamid und bevorzugt Ketone, wie Aceton und Methylethylketon. Die Herstellung von Oligomeren, die keine Hydroxylgruppen-haltigen Komponenten eingebaut enthalten, erfolgt durch Umsetzung der Amingruppen-haltigen Komponenten B) mit den Diisocyanaten A) bei einer Temperatur im Bereich von etwa 0 bis 60°C, bevorzugt 10 bis 50 °C. Neben den zuvor genannten Lösungsmitteln kann die Herstellung von Oligomeren, die keine Hydroxylgruppen-haltigen Komponenten eingebaut enthalten, auch in Wasser, C₁- bis C₄-Alkoholen, wie Methanol, n-Propanol, iso-Propanol, n-Butanol und bevorzugt in Ethanol und Ethanol-Wasser-Gemischen erfolgen. Vorzugsweise erfolgt die Reaktion unter einer Inertgasatmosphäre, wie z. B. unter Stickstoff. Des Weiteren erfolgt die Reaktion vorzugsweise bei Umgebungsdruck oder unter erhöhtem Druck. Die Komponenten werden bevorzugt in solchen Mengen eingesetzt, dass das Verhältnis von NCO-Äquivalent der Verbindungen der Komponente A) zu Äquivalent aktives Wasserstoffatom der Komponenten B) in einem Bereich von etwa 0,3:1 bis 1,1:1, bevorzugt 0,4:1 bis 0,9:1, liegt.

Die Herstellung von Oligomeren aus den Komponenten A), B) und C) erfolgt bevorzugt durch Umsetzung wenigstens eines Reaktionsproduktes aus den Komponenten A) und B) mit wenigstens einer Carbonsäure C).

Die Herstellung erfolgt vorzugsweise ohne Zusatz eines Lösungsmittels. Die Reaktionstemperatur liegt bevorzugt in einem Bereich von etwa 100 bis 250 °C, insbesondere 150 bis 220 °C. Die Entfernung des bei der Reaktion gebildeten Reaktionswassers erfolgt nach üblichen, dem Fachmann bekannten Methoden, wie z. B. durch Abdestillieren. Die Reaktion kann bei Normaldruck oder vorzugsweise bei verringertem Druck erfolgen. Die Polykondensationsreaktion kann durch Einsatz von Katalysatoren in den hierbei üblichen Mengen beschleunigt werden. Geeignete Katalysatoren sind z. B. Schwefelsäure, Phosphorsäure, Alkyl- und Arylsulfonsäuren, saure Ionenaustauscher, Tetrabutyltitanat etc.

Die Herstellung von Oligomeren aus den Komponenten A), B) und C) kann auch durch Umsetzung wenigstens eines Umsetzungsproduktes aus den Komponenten A) und B) mit einem Derivat einer Säure C) erfolgen. Geeignete Derivate sind z. B. die Säurehalogenide, -anhydride und die -ester mit C₁- bis C₄-Alkanolen.

Bei der Herstellung der Oligomeren aus den Komponenten A), B) und C) wird das Molmengenverhältnis von Umsetzungsprodukt aus A) und B) zu Dicarbonsäure bzw. Hydroxycarbonsäure C) vorzugsweise so gewählt, dass die resultierenden Oligomere im Wesentlichen keine freien Carbonsäuregruppen aufweisen.

Vorzugsweise enthalten die kosmetischen Mittel wenigstens ein Oligomer aus den Komponenten A) und B1). Bei der Herstellung dieser Oligomere liegt das Verhältnis von NCO-Äquivalent der Verbindungen der Komponente A) zu Äquivalent aktives Wasserstoffatom der Verbindungen der Komponente B1) in einem Bereich von etwa 0,3:1 bis 0,9:1. Bevorzugt weisen die Oligomere aus den Komponenten A) und B1) ein Molekulargewicht im Bereich von etwa 500 bis 5000, besonders bevorzugt 600 bis 3000, insbesondere 700 bis 2000, auf.

Vorzugsweise enthalten die kosmetischen Mittel wenigstens ein Oligomer aus den Komponenten A) und B2). Bei der Herstellung dieser Oligomere liegt das Verhältnis von NCO-Äquivalent der Verbindungen der Komponente A) zu Äquivalent aktives Wasserstoffatom der Komponenten B2) in einem Bereich von etwa 0,5:1 bis 1:1, bevorzugt 0,5:1 bis 0,95:1, besonders bevorzugt 0,5:1 bis 0,8:1. Bevorzugt weisen die Oligomeren aus den Komponenten A) und B2) ein Molekulargewicht im.Bereich von 500 bis 5000, besonders bevorzugt 600 bis 3000 auf.

Vorzugsweise enthalten die kosmetischen Mittel wenigstens ein Oligomer aus den Komponenten A) und B3). Bei der Herstellung dieser Oligomere liegt das Verhältnis von NCO-Äquivalent der Verbindungen der Komponente A) zu Äquivalent aktives Wasserstoffatom der Komponenten B3) in einem Bereich von etwa 0,5:1 bis 0,9:1. Diese Polysiloxane weisen vorzugsweise ein zahlenmittleres Molekulargewicht im Bereich von etwa 300 bis 5000, bevorzugt 400 bis 3000, auf.

Vorzugsweise enthalten die kosmetischen Mittel wenigstens ein Oligomer aus den Komponenten A) und B4). Bei der Herstellung dieser Oligomere liegt das Verhältnis von NCO-Äquivalent der Verbindungen der Komponente A) zu Äquivalent aktives Wasserstoffatom der Komponenten B4) in einem Bereich von etwa 0,4:1 bis 1:1, bevorzugt 0,4:1 bis 0,95:1, besonders bevorzugt 0,4:1 bis 0,9:1. Bevorzugt liegt das Molekulargewicht der Oligomeren aus den Komponenten A) und B4) in einem Bereich von 500 bis 7000, besonders bevorzugt 600 bis 6000, insbesondere 700 bis 5000.

Vorzugsweise enthalten die kosmetischen Mittel wenigstens ein Oligomer aus den Komponenten A), B), besonders bevorzugt B1), und C). Diese Reaktionsprodukte haben ein Molekulargewicht im Bereich von 500 bevorzugt 600, insbesondere 700, bis 10000.

Ein weiterer Gegenstand der Erfindung ist ein Oligomer, das mindestens ein Diisocyanat A) und mindestens eine Komponente B4), wie zuvor beschrieben, eingebaut enthält.

Ein weiterer Gegenstand der Erfindung ist ein Oligomer, das mindestens ein Diisocyananat A), mindestens eine Komponente B) und mindestens eine Dicarbonsäure und/oder Hydroxycarbonsäure C), wie zuvor beschriebene, eingebaut enthält.

Nach einer bevorzugten Ausführungsform enthalten die erfindungsgemäßen kosmetischen Mittel wenigstens ein Oligomer, wie zuvor beschrieben, in Form einer separaten Komponente.

Nach einer bevorzugten Ausführungsform liegen die erfindungsgemäßen kosmetischen Mittel, welche die Oligomere als separate Komponente enthalten, in Form eines Haarbehandlungsmittels vor. Dazu zählen z. B. Haarsprays, Schaumfestiger, Haarmousse, Haargel und Shampoos. Geeignete Komponenten zur Formulierung von Haarbehandlungsmitteln werden im Folgenden für Haarbehandlungsmittel auf Basis von Polymeren, die die Oligomere einpolymerisiert enthalten, ausführlich beschrieben. Auf die dort genannten Inhaltsstoffe und Formulierungen wird in vollem Umfang Bezug genommen. Bevorzugt enthalten die Haarbehandlungsmittel die Oligomere in einer Menge im Bereich von etwa 0,01 bis 20 Gew.-%, bevorzugt 0,1 bis 15 Gew.-%, bezogen auf die Gesamtmenge des Mittels. Im Allgemeinen weisen Haarkosmetika auf Basis der zuvor beschriebenen Oligomere bessere Filmeigenschaften, wie z. B. eine verringerte Klebrigkeit, auf als entsprechende Produkte ohne diese Additive. Im Allgemeinen wird mit den erfindungsgemäßen Mitteln eine bessere Flexibilität erzielt als mit herkömmlichen Mitteln. Die mit den erfindungsgemäßen Mitteln behandelten Haare weisen somit im Allgemeinen gute Geschmeidigkeit und/oder Kämmbarkeit auf.

Zur Formulierung von Haarfestigern werden bevorzugt Oligomere aus den Komponenten A) und B1) eingesetzt.

Nach einer weiteren bevorzugten Ausführungsform liegen die erfindungsgemäßen kosmetischen Mittel in Form eines ölhaltigen oder fetthaltigen kosmetischen Präparates vor. Dazu zählen z. B. Cremes, Mascara, Augen-Make-up, Gesichts-Make-up, kosmetische Öle, Babyöl, Badeöl, Make-up-Entferner, Hautfeuchthaltemittel, Sonnenschutzmittel, Lippenpflegemittel, wasserfreie Handwaschmittel und medizinische Salben.

Die erfindungsgemäßen ölhaltigen bzw. fetthaltigen kosmetischen mittel weisen z. B. eine Öl- bzw. Fettkomponente auf, die ausgewählt ist unter: Kohlenwasserstoffen geringer Polarität, wie Mineralölen; linearen gesättigten Kohlenwasserstoffen, wie Tetradecan, Hexadecan und Octadecan ; cyclischen Kohlenwasserstoffen, wie Decahydronaphthalin; verzweigten Kohlenwasserstoffen; Estern, bevorzugt Estern von Fettsäuren, wie z. B. die Ester von C₁- bis C₂₄-Monoalkoholen mit C₁- bis C₂₂-Monocarbonsäuren, wie Isopropylisostearat, n-Propylmyristat, iso-Propylmyristat, n-Propylpalmitat, iso-Propylpalmitat, Hexacosanylpalmitat, Octacosanylpalmitat, Triacontanylpalmitat, Dotriacontanylpalmitat, Tetratriacontanylpalmitat, Hexancosanylstearat, Octacosanylstearat, Triacontanylstearat, Dotriacontanylstearat, Tetratriacontanylstearat; Salicylaten, wie C₁- bis C₁₀-Salicylaten, z. B. Octylsalicylat; Benzoatestern, wie C₁₀- bis C₁₅-Alkylbenzoaten, Benzylbenzoat; anderen kosmetischen Estern, wie Fettsäuretriglyceriden, Propylenglykolmonolaurat, Polyethylenglykolmonolaurat, Castoröl, C₁₀- bis C₁₅-Alkyllactaten, etc. Die erfindungsgemäßen ölhaltigen bzw. fetthaltigen kosmetischen Mittel enthalten diese Komponenten im Allgemeinen in einer Menge von mindestens 0,1, bevorzugt mindestens 0,2, insbesondere mindestens 0,5 Gew.-%. Geeignet sind z. B. Mengen von etwa 0,1 bis 99,9 Gew.-%, bevorzugt 1 bis 99,9 Gew.-%, besonders bevorzugt 10 bis 90 Gew.-%, insbesondere 20 bis 80 Gew.-%, bezogen auf das Gesamtgewicht des Mittels.

Die erfindungsgemäßen ölhaltigen bzw. fetthaltigen kosmetischen Mittel enthalten die zuvor beschriebenen Oligomere oder deren Reaktionsprodukte im Allgemeinen in einer Menge von etwa 0,1 bis 50 Gew.-%, bevorzugt 0,2 bis 40 Gew.-%, besonders bevorzugt 0,2 bis 30 Gew.-%, insbesondere 0,5 bis 10 Gew.-%, bezogen auf die Gesamtmenge des Mittels.

Weiterhin können die ölhaltigen bzw. fetthaltigen kosmetischen Mittel Hilfs- und/oder Zusatzstoffe, wie Emulgatoren, Überfettungsmittel, Stabilisatoren, Wachse, Konsistenzgeber, Verdikkungsmittel, Siliconverbindungen, biogene Wirkstoffe, Filmbildner, Konservierungsmittel, Hydrotrope, Solubilisatoren, UV-Adsorber, Farb- und Duftstoffe enthalten.

Vorzugsweise wird zur Herstellung der erfindungsgemäßen öl- bzw. fetthaltigen kosmetischen Mittel ein Oligomer eingesetzt, das wenigstens ein Diisocyanat A) und wenigstens eine Komponente B), die ausgewählt ist unter den Komponenten B2) bis B4), eingebaut enthält. Vorzugsweise wird zur Herstellung der öl- bzw. fetthaltigen Mittel weiterhin ein Oligomer aus den Komponenten A), B), bevorzugt B1), und C) eingesetzt.

Die erfindungsgemäßen öl- bzw. fetthaltigen kosmetischen Produkte auf Basis der zuvor beschriebenen Oligomere lassen sich im Allgemeinen bezüglich ihrer rheologischen Eigenschaften bzw. ihrer Konsistenz in einem weiten Bereich einstellen. Je nach Grundkonsistenz des kosmetischen Mittels können die Eigenschaften im Allgemeinen in Abhängigkeit von der Einsatzmenge des Oligomers von einer dünnflüssigen bis zu einer festen Konsistenz variiert werden. Vorteilhafterweise lassen sich somit hautkosmetische Produkte formulieren, die einen hohen Anteil an dünnflüssigen öl- bzw. fetthaltigen Komponenten aufweisen.

Vorteilhafterweise eignen sich die beschriebenen Oligomere zur Formulierung von Gelen. Unter "Gel" versteht man im Allgemeinen eine Formulierung, die eine höhere Viskosität als eine Flüssigkeit aufweist und die selbsttragend ist, d. h. eine ihr verliehene Form ohne formstabilisierende Umhüllung behält. Zur Formulierung von Gelen eignen sich im Allgemeinen alle zuvor genannten Ölkomponenten, die bei Umgebungstemperatur flüssig sind. Vorteilhafterweise sind Gele auf Basis der zuvor beschriebenen Oligomere im Allgemeinen transparent. Sie können mit üblichen Zusatzstoffen zu erfindungsgemäßen kosmetischen Mitteln, wie z. B. Lippenpflegemitteln, Desodorantien, Antiperspirantien, Make-ups, etc., formuliert werden. Vorteilhafterweise können die beschriebenen Oligomere auch zur Herstellung nicht-kosmetischer Produkte auf Gelbasis eingesetzt werden. Dazu zählen z. B. Autowachse und -polituren, Kerzen, Möbelpolituren, Lederpflegemittel, Metallreiniger, Haushaltsreiniger, etc.

Vorteilhafterweise eignen sich die beschriebenen Oligomere auch zur Herstellung üblicher O/W- und W/O-Formulierungen, wie z. B. Cremes, wobei sie im Allgemeinen sowohl in der Ölphase als auch in der Wasserphase eingesetzt werden können.

Ein weiterer Gegenstand der Erfindung ist die Verwendung von Oligomeren und deren Reaktionsprodukten, wie zuvor beschrieben, als Komponente pharmazeutischer und kosmetischer Zubereitungen, bevorzugt in kosmetischen Zubereitungen zur Behandlung der Haut oder der Haare, zur Modifizierung der rheologischen Eigenschaften von Zusammensetzungen auf Basis von Verbindungen geringer Polarität sowie als Zwischenprodukte zur Herstellung wasserlöslicher oder wasserdispergierbarer Polyurethane.

Ein weiterer Gegenstand der Erfindung ist die Verwendung von Oligomeren und deren Reaktionsprodukten, wie zuvor beschrieben, als oder in Beschichtungsmittel(n) und als oder in Behandlungsmittel(n) für nichtsaugfähige Oberflächen, bevorzugt Metalle, Kunststoffe, Textilsynthesefasern und Glas, und für saugfähige Oberflächen, bevorzugt Holz, Papier, Baumwolle und Leder.

Die genannten Oligomere eignen sich insbesondere als Verdickungsmittel für Flüssigkeiten niederer Polarität, vorzugsweise Öle. Bevorzugt werden Oligomere als Verdicker für Öle eingesetzt, die einen auf das Gesamtgewicht bezogenen Anteil von Urethan- und/oder Harnstoffgruppen von höchstens 5 Gew.-% aufweisen. Diese Komponenten weisen vorzugsweise eine hohe Verträglichkeit mit nicht-siliconhaltigen Ölen auf. Sie sind im Allgemeinen in Siliconölen, nicht-siliconhaltigen Ölen oder Mischungen davon löslich. Vorteilhafterweise sind die dabei erhaltenen Lösungen im Allgemeinen klar. Vorteilhafterweise lassen sich klare kosmetische Formulieren z. B. leichter anfärben als bereits gefärbte. Die zuvor beschriebenen Oligomere und deren Reaktionsprodukte eignen sich vorzugsweise für die Verwendung in Personal-Care-Produkte, wie beispielsweise kosmetischen Mitteln, z. B. Augen-Make-up, Gesichts-Make-up, Babyöl, Badeöl, Make-up-Entferner, Hautfeuchthaltemitteln, Sonnenschutzmitteln, Lippenpflegemitteln, wasserfreien Handwaschmitteln, kosmetischen Gelen, Salben, Wachsen, medizinischen Salben, Parfums und Suppositorien. Sie eignen sich weiterhin vorteilhafterweise zur Formulierung haarkosmetischer Produkte, wie Haarsprays, Schaumfestiger, Haarmousse, Haargel und Shampoos. Sie eignen sich weiterhin bevorzugt für eine Verwendung in der dekorativen Kosmetik, insbesondere in Mascara und Lidschatten. Ferner können die zuvor beschriebenen Oligomere und deren Reaktionsprodukte vorteilhaft in Haushaltsprodukten, wie Autowachsen und -polituren, Kerzen, Möbelpolituren, Metallreinigungsmitteln und Metallpolituren, Haushaltsreinigern, Farbentfernern und Trägermaterialien für Insektizide eingesetzt werden. Sie eignen sich weiterhin für die Anwendung in technischen bzw. industriellen Produkte, wie beispielsweise in Kraftstoffen, Fetten, Lötfetten, Rostschutzmitteln und Tintenstrahldruckerpatronen.

Die zuvor beschriebenen Oligomere weisen, im Gegensatz zu den beschriebenen Polymeren, immer zwingend wenigstens zwei funktionelle Gruppen, die ausgewählt sind unter Hydroxyl-, primären und sekundären Aminogruppen, auf. Sie eignen sich in vorteilhafter Weise zum Aufbau segmentierter Polyurethane, die diese Oligomere in eingebauter Form als Wiederholungseinheit aufweisen.

Ein weiterer Gegenstand der Erfindung sind somit segmentierte Polyurethane, die mindestens ein Oligomer, wie zuvor beschrieben, wenigstens einen niedermolekularen Kettenverlängerer, wenigstens eine Verbindung mit mindestens einer ionogenen und/oder ionischen (hydrophilen bzw. dispergieraktiven) Gruppe und wenigstens ein Diisocyanat eingebaut enthalten.

Die Polyurethane auf Basis der zuvor beschriebenen Oligomere sind wasserlöslich oder wasserdispergierbar.

Die zuvor beschriebenen Oligomere eignen sich insbesondere als Komponente zur Herstellung wasserlöslicher oder wasserdispergierbarer Polyurethane. Ein weiterer Gegenstand der Erfindung sind wasserlösliche oder wasserdispergierbare Polyurethane aus:
a) mindestens einem Oligomer, wie zuvor beschrieben,
b) mindestens einer Verbindung mit einem Molekulargewicht im Bereich von 56 bis 600, die zwei aktive Wasserstoffatome pro Molekül enthält,
c) mindestens einer Verbindung, die zwei aktive Wasserstoffatome und mindestens eine ionogene und/oder ionische Gruppe pro Molekül aufweist,
d) gegebenenfalls mindestens einem Polymerisat mit mindestens zwei aktiven Wasserstoffatomen pro Molekül,
e) mindestens einem Diisocyanat.

Die erfindungsgemäßen Polyurethane enthalten wenigstens eines der zuvor beschriebenen Oligomere als Komponente a) in eingebauter (einpolymerisierter) Form.

Bei der Komponente b) handelt es sich bevorzugt um Diole, Diamine, Aminoalkohole, und Mischungen davon. Das Molekulargewicht dieser Verbindungen liegt vorzugsweise in einem Bereich von etwa 56 bis 500. Gewünschtenfalls können bis zu 3 Mol-% der genannten Verbindungen durch Triole oder Triamine ersetzt sein.

Bevorzugt werden als Komponente b) Diole eingesetzt. Brauchbare Diole sind z. B. Ethylenglykol, Propylenglykol, Butylenglykol, Neopentylglykol, Cyclohexandimethylol, Di-, Tri-, Tetra-, Penta- oder Hexaethylenglykol und Mischungen davon. Bevorzugt werden Neopentylglykol, Glycerinmonostearat und/oder Cyclohexandimethylol eingesetzt.

Geeignete Aminoalkohole b) sind z. B. 2-Aminoethanol, 2-(N-Methylamino)ethanol, 3-Aminopropanol, 4-Aminobutanol, 1-Ethylaminobutan-2-ol, 2-Amino-2-methyl-1-propanol und 4-Methyl-4-aminopentan-2-ol.

Geeignete Diamine b) sind z. B. Ethylendiamin, Propylendiamin, 1,4-Diaminobutan, 1,5-Diaminopentan und 1,6-Diaminohexan.

Geeignete Diamine b) sind auch Diamine der Formel
R^{a}-NH-(CH₂)₂₋₃-NH₂, wobei R^{a} für C₈- bis C₂₂-Alkyl oder C₈- bis C₂₂-Alkenyl steht, wobei der Alkenylrest 1,2 oder 3 nicht benachbarte Doppelbindungen aufweisen kann. Das Molekulargewicht dieser Diamine b) liegt vorzugsweise in einem Bereich von etwa 160 bis 400.

Weiterhin geeignete Diamine b), die üblicherweise als Kettenverlängerer eingesetzt werden, sind z. B. Hexamethylendiamin, Piperazin, 1,2-Diaminocyclohexan, 1,3-Diaminocyclohexan, 1,4-Diaminocyclohexan, Neopentandiamin und 4,4'-Diaminodicyclohexylmethan.

Geeignete Verbindungen c) weisen zwei aktive Wasserstoffatome und mindestens eine ionogene und/oder ionische Gruppe pro Molekül auf, wobei es sich um anionogene, anionische, kationogene oder kationische Gruppen handelt.

Bevorzugte Verbindungen c) mit zwei aktiven Wasserstoffatomen und mindestens einer anionogenen und/oder anionischen Gruppe pro Molekül sind z. B. Verbindungen mit Carboxylat- und/oder Sulfonatgruppen. Als Komponente c) sind 2,2-Hydroxymethyl-alkylcarbonsäuren, wie Dimethylolpropansäure, und Mischungen, die 2,2-Hydroxymethyl-alkylcarbonsäuren, wie Dimethylolpropansäure, enthalten, besonders bevorzugt.

Geeignete Diamine und/oder Diole c) mit anionogenen oder anionischen Gruppen sind Verbindungen der Formel 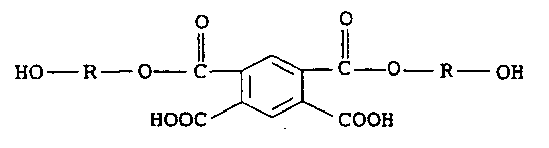 und/oder 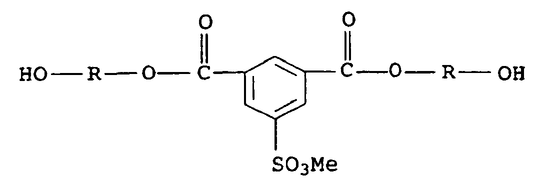 worin R jeweils für eine C₂-C₁₈-Alkylengruppe steht und Me für Na oder K steht.

Als Komponente c) brauchbar sind auch Verbindungen der Formel

H₂N(CH₂)_{w}-NH-(CH₂)ₓ-COO-M⁺

H₂N(CH₂)_{w}-NH-(CH₂)ₓ-SO₃⁻M⁺

worin w und x unabhängig voneinander für eine ganze Zahl von 1 bis 8, insbesondere 1 bis 6, stehen und M für Li, Na oder K steht, und Verbindungen der Formel

H₂N(CH₂CH₂O)_{y}(CH₂CH(CH₃)O)_{z}(CH₂)_{w}-NH-(CH₂)ₓ-SO₃-M⁺

worin w und x die zuvor angegebenen Bedeutungen besitzen, y und z unabhängig voneinander für eine ganze Zahl von 0 bis 50 stehen, wobei wenigstens eine der beiden Variablen y oder z > 0 ist. Die Reihenfolge der Alkylenoxideinheiten ist dabei beliebig. Die zuletzt genannten Verbindungen weisen vorzugsweise ein zahlenmittleres Molekulargewicht im Bereich von etwa 400 bis 3 000 auf. Eine geeignete Verbindung dieses Typs ist z. B. Poly ESP 520 der Fa. Raschig.

Die Polyurethane können auch Verbindungen c) eingebaut enthalten, die zwei aktive Wasserstoffatome und mindestens eine kationogene und/oder kationische Gruppe, bevorzugt mindestens eine stickstoffhaltige Gruppe, pro Molekül aufweisen. Bevorzugt handelt es sich bei der stickstoffhaltigen Gruppe um eine tertiäre Aminogruppe oder eine quaternäre Ammoniumgruppe. Bevorzugt sind z. B. Verbindungen der allgemeinen Formeln 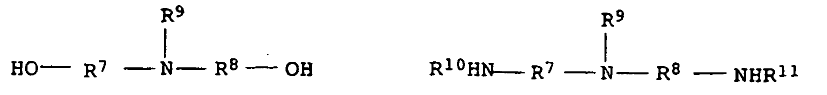 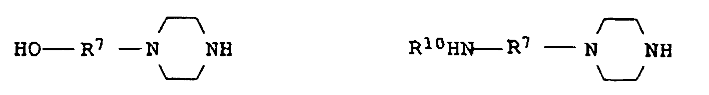 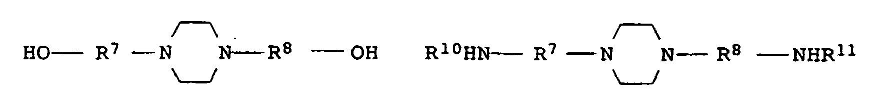 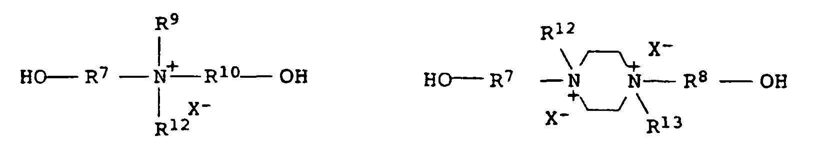 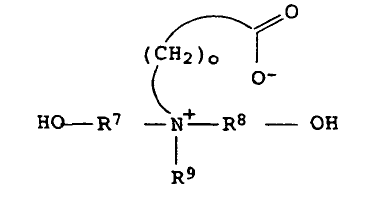 worin
- R⁷ und R⁸, die gleich oder verschieden sein können, für C₂-C₉-Alkylen stehen,
- R⁹, R¹² und R¹³, die gleich oder verschieden sein können, für C₁-C₆-Alkyl, Phenyl oder Phenyl-C₁-C₄-alkyl stehen, R¹⁰ und R¹¹, die gleich oder verschieden sein können, für H oder C₁-C₆-Alkyl stehen, o für 1, 2 oder 3 steht,
X^{⊖} für Chlorid, Bromid, Jodid, C₁-C₆-Alkylsulfat oder SO₄²⁻/₂ steht.

Besonders bevorzugt sind N-(C₁- bis C₆-alkyl)diethanolamine, wie Methyldiethanolamin, und N-Alkyldialkylentriamine, wie N-Methyldipropylentriamin. Diese werden vorzugsweise in Kombination mit Dimethylolpropansäure als Komponente c) eingesetzt.

Als Komponente c) eignen sich auch Gemische, die zwei oder mehrere der zuvor genannten Verbindungen mit anionischen und/oder anionogenen Gruppen, zwei oder mehrere der zuvor genannten Verbindungen mit kationischen und/oder kationogenen Gruppen oder Gemische, die mindestens eine der zuvor genannten Verbindungen mit anionischen oder anionogenen Gruppen und mindestens eine der zuvor genannten Verbindungen mit kationischen oder kationogenen Gruppen enthalten. Bevorzugt werden z. B. Gemische eingesetzt, die Dimethylolpropansäure und N-Methyldiethanolamin enthalten. Nach einer bevorzugten Ausführungsform enthalten die Polyurethane überwiegend oder ausschließlich anionogene und/oder anionische Gruppen als ionogene und/oder ionische Gruppen. Nach einer weiteren bevorzugten Ausführungsform enthalten die Polyurethane überwiegend oder ausschließlich kationogene und/oder kationische Gruppen als ionogene und/oder ionische Gruppen. Bevorzugt enthalten die Polyurethane somit eine Komponente c) eingebaut, die überwiegend, bevorzugt zu mindestens 80 Gew.-%, insbesondere zu mindestens 90 Gew.-%, bezogen auf die Gesamtmenge der Komponente c), entweder anionogene (anionische) Verbindungen oder kationogene (kationische) Verbindungen umfasst.

Bei der Komponente d) handelt es sich bevorzugt um ein Polymerisat mit einem zahlenmittleren Molekulargewicht im Bereich von etwa 300 bis 5000, bevorzugt etwa 400 bis 4000, insbesondere 500 bis 3000. Brauchbare Polymerisate d) sind z. B. Polyesterdiole, Polyetherole, Polysiloxane und Mischungen davon. Polyetherole sind vorzugsweise Polyalkylenglykole, z. B. Polyethylenglykole, Polypropylenglykole, Polytetrahydrofurane etc., Copolymerisate aus Ethylenoxid und Propylenoxid oder Blockcopolymerisate aus Ethylenoxid, Propylenoxid und Butylenoxid, die die Alkylenoxideinheiten statistisch verteilt oder in Form von Blöcken einpolymerisiert enthalten. Geeignet sind auch α,ω-Diaminopolyether, die durch Aminierung von Polyalkylenoxiden mit Ammoniak herstellbar sind. Vorzugsweise werden als Komponente d) Polyesterdiole und Mischungen, die diese enthalten, eingesetzt.

Geeignete Polytetrahydrofurane d) können durch kationische Polymerisation von Tetrahydrofuran in Gegenwart von sauren Katalysatoren, wie z. B. Schwefelsäure oder Fluoroschwefelsäure, hergestellt werden. Derartige Herstellungsverfahren sind dem Fachmann bekannt.

Bevorzugte Polyesterdiole d) weisen ein zahlenmittleres Molekulargewicht im Bereich von etwa 400 bis 5000, bevorzugt 500 bis 3000, insbesondere 600 bis 2000, auf.

Als Polyesterdiole kommen alle diejenigen in Betracht, die üblicherweise zur Herstellung von Polyurethanen eingesetzt werden, insbesondere solche auf Basis aromatischer Dicarbonsäuren, wie Terephthalsäure, Isophthalsäure, Phthalsäure, Na- oder K-Sulfoisophthalsäure etc., aliphatischer Dicarbonsäuren, wie Adipinsäure oder Bernsteinsäure etc., und cycloaliphatischer Dicarbonsäuren, wie 1,2-, 1,3- oder 1,4-Cyclohexandicarbonsäure. Als Diole kommen insbesondere aliphatische Diole in Betracht, wie Ethylenglykol, Propylenglykol, 1,6-Hexandiol, Neopentylglykol, Diethylenglykol, Polyethylenglykole, Polypropylenglykole, 1,4-Dimethylolcyclohexan.

Bevorzugt sind Polyesterdiole auf Basis von aromatischen und aliphatischen Dicarbonsäuren und aliphatischen Diolen, insbesondere solche, bei denen die aromatische Dicarbonsäure 10 bis 95 Mol-%, insbesondere 40 bis 90 Mol-% des gesamten Dicarbonsäureanteils (Rest aliphatische Dicarbonsäuren) ausmacht.

Besonders bevorzugte Polyesterdiole sind die Umsetzungsprodukte aus Phthalsäure/Diethylenglykol, Isophthalsäure/1,4-Butandiol, Isophthalsäure/Adipinsäure/1,6-Hexandiol, 5-NaSO₃-Isophthalsäure/Phthalsäure/Adipinsäure/1,6-Hexandiol, Adipinsäure/Ethylenglykol, Isophthalsäure/Adipinsäure/Neopentylglykol, Isophthalsäure/Adipinsäure/Neopentylglykol/Diethylenglykol/Dimethylolcyclohexan und 5-NaSO₃-Isophthalsäure/Isophthalsäure/Adipinsäure/Neopentylglykol/Diethylenglykol/Dimethylolcyclohexan, Isophthalsäure/Adipinsäure, Neopentylglykol/Dimethylolcyclohexan.

Bevorzugt als Komponente d) sind weiterhin die zuvor bereits als Komponente B4) genannten Polyesterdiole auf Basis von linearen oder verzweigten, C₈- bis C₃₀-Di- oder Polycarbonsäuren und C₈₋bis C₃₀-Hydroxycarbonsäuren. Bevorzugte Carbonsäuren und Hydroxycarbonsäuren sind z. B. Acelainsäure, Dodecandisäure, Korksäure, Pimelinsäure, Sebacinsäure, Tetradecandisäure, Citronensäure, Ricinolsäure, Hydroxystearinsäure und Gemische davon. Als Diolkomponente zur Herstellung dieser Polyesterdiole werden vorzugsweise 1,4-Butandiol, 1,5-Pentandiol, 1,6-Hexandiol, Neopentylglykol, 1,4-Dimethylolcyclohexan, Diethylenglykol, Glycerinmonostearat und Gemische davon eingesetzt.

Bevorzugt als Komponente d) sind weiterhin die zuvor genannten Polysiloxane B3). Vorzugsweise enthält nur eine der Komponenten a) oder d) ein Polysiloxan.

Weiterhin bevorzugt handelt es sich bei der Komponente d) um ein Diaminopolyethersiloxan der Formel IV, das ausgewählt ist unter
- Polysiloxanen mit Wiederholungseinheiten der allgemeinen Formel IV.I 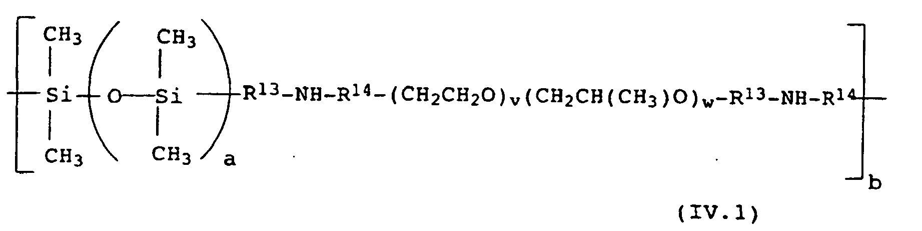 worin
   - a: für eine ganze Zahl von 0 bis 100 steht,
   - b: für eine ganze Zahl von 1 bis 8 steht,
   - R¹³ und R¹⁴: unabhängig voneinander für C₁- bis C₈-Alkylen stehen,
   die Reihenfolge der Alkylenoxideinheiten beliebig ist und v und w unabhängig voneinander für eine ganze Zahl von 0 bis 200 stehen, wobei die Summe aus v und w > 0 ist,
- Polysiloxanen der allgemeinen Formel IV.2 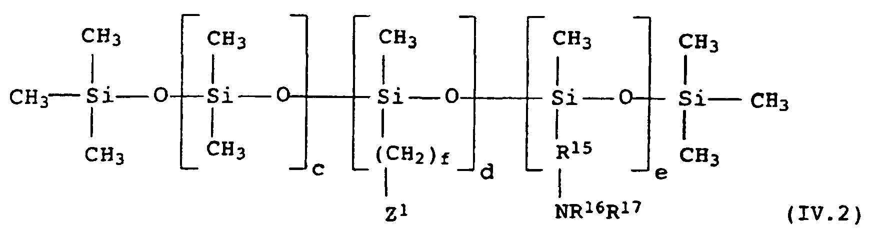 worin
   - R¹⁵: für einen C₁- bis C₈-Alkylenrest steht,
   - R¹⁶ und R¹⁷: unabhängig voneinander für Wasserstoff, C₁- bis C₈-Alkyl oder C₅- bis C₈-Cycloalkyl stehen,
   die Reihenfolge der Siloxaneinheiten beliebig ist, c, d und e unabhängig voneinander für 0 bis 100 stehen, wobei die Summe aus c, d und e mindestens 3 ist,
   - f: für eine ganze Zahl von 2 bis 8 steht,
   - Z¹: für einen Rest der Formel V

   -R¹⁸-(CH₂CH₂O)_{g}(CH₂CH(CH₃)O)ₕ-R¹⁹ (V)
   steht, worin
   die Reihenfolge der Alkylenoxideinheiten beliebig ist und g und h unabhängig voneinander für eine ganze Zahl von 0 bis 200 stehen, wobei die Summe aus g und h > 0 ist,
   - R¹⁸: für einen C₁- bis C₈-Alkylenrest steht und
   - R¹⁹: für Wasserstoff oder einen C₁- bis C₈-Alkylrest steht,
und Mischungen davon.

Bevorzugt stehen in der Formel IV.1 R¹³ und R¹⁴ unabhängig voneinander für einen C₂- bis C₄-Alkylenrest. Insbesondere stehen R¹³ und R¹⁴ unabhängig voneinander für einen C₂- bis C₃-Alkylenrest.

Vorzugsweise liegt das Molekulargewicht der Verbindung der Formel IV.1 in einem Bereich von etwa 300 bis 100000 liegt.

Vorzugsweise steht in der Formel IV.1 a für eine ganze Zahl von 1 bis 20, wie z. B. 2 bis 10.

Bevorzugt liegt die Gesamtzahl der Alkylenoxideinheiten der Verbindung der Formel IV.1, d. h. die Summe aus v und w, in einem Bereich von etwa 3 bis 200, bevorzugt 5 bis 180.

Bevorzugt sind die Endgruppen der Polysiloxane mit Wiederholungseinheiten der allgemeinen Formel IV.1 ausgewählt unter (CH₃)₃SiO, H, C₁- bis C₈-Alkyl und Mischungen davon.

Geeignete alkoxylierte Siloxanamine der Formel IV-1 sind z. B. in der WO-A-97/32917 beschrieben, auf die hier in vollem Umfang Bezug genommen wird. Kommerziell erhältliche Verbindungen sind z. B. die Silsoft®-Marken der Fa. Witco, z. B. Silsoft® A-843.

Bevorzugt steht in der Formel IV.2 der Rest R¹⁵ für einen C₂- bis C₄-Alkylenrest.

Bevorzugt stehen in der Formel IV.2 R¹⁶ und R¹⁷ unabhängig voneinander für Wasserstoff oder C₁- bis C₄-Alkyl.

Vorzugsweise wird die Summe aus c, d und e so gewählt, dass das Molekulargewicht der Verbindung der Formel IV.2 in einem Bereich von etwa 300 bis 100000, bevorzugt 500 bis 50000, liegt.

Bevorzugt liegt die Gesamtzahl der Alkylenoxideinheiten des Restes der Formel V, d. h. die Summe aus g und h, in einem Bereich von etwa 3 bis 200, bevorzugt 5 bis 80.

Bevorzugt steht in der Formel V der Rest R¹⁸ für C₂- bis C₄-Alkyl.

Bevorzugt steht in der Formel V der Rest R¹⁹ für Wasserstoff oder C₁- bis C₄-Alkyl.

Eine geeignete Verbindung der Formel IV.2 ist z. B. Silsoft® A-858 der Fa. Witco.

Bei der Komponente e) handelt es sich um übliche aliphatische, cycloaliphatische und/oder aromatische Diisocyanate, wie Tetramethylendiisocyanat, Hexamethylendiisocyanat, Methylendiphenyldiisocyanat, 2,4- und 2,6-Toluylendiisocyanat und deren Isomerengemische, o-, m- und p-Xylylendiisocyanat, 1,5-Naphthylendiisocyanat, 1,4-Cyclohexylendiisocyanat, Dicyclohexylmethandiisocyanat und Mischungen davon, insbesondere Isophorondiisocyanat, Hexamethylendiisocyanat und/oder Dicyclohexylmethandiisocyanat. Gewünschtenfalls können bis zu 3 Mol-% der genannten Verbindungen durch Triisocyanate ersetzt sein.

Die Herstellung der erfindungsgemäßen Polyurethane erfolgt durch Umsetzung wenigstens eines Oligomers a) und der Verbindungen der Komponenten b) und c) sowie gegebenenfalls d) mit der Diisocyanatkomponente e). Dabei liegt das Verhältnis von NCO-Äquivalent der Komponente e) zu Äquivalent aktives Wasserstoffatom der Komponenten a) bis d) im Allgemeinen in einem Bereich von etwa 0,6:1 bis 1,4:1, bevorzugt 0,8:1 bis 1,2:1, insbesondere 0,9:1 bis 1,1:1. Die Reaktion kann vorzugsweise ohne Lösungsmittel oder in einem geeigneten inerten Lösungsmittel oder Lösungsmittelgemisch erfolgen. Geeignete Lösungsmittel sind aprotisch polare Lösungsmittel, z. B. Tetrahydrofuran, Essigsäureethylester, N-Methylpyrrolidon, Dimethylformamid und bevorzugt Ketone, wie Aceton und Methylethylketon. Vorzugsweise erfolgt die Reaktion unter einer Inertgasatmosphäre, wie z. B. unter Stickstoff. Des Weiteren erfolgt die Reaktion vorzugsweise bei Umgebungsdruck oder unter erhöhtem Druck. Enthalten die Komponenten a) bis d) Hydroxylgruppen-haltige Verbindungen, so liegt die Reaktionstemperatur bevorzugt in einem Bereich von etwa 50 bis 150 °C. Die Reaktion erfolgt dann vorzugsweise in einem Lösungsmittel oder Lösungsmittelgemisch, welches keine aktiven Wasserstoffatome aufweist. Bevorzugt werden Ketone, wie Aceton, Methylethylketon und Gemische davon eingesetzt. Werden als Komponenten a) bis d) ausschließlich oder überwiegend Verbindungen eingesetzt, die als gegenüber Isocyanatgruppen reaktiven Gruppen primäre und/oder sekundäre Aminogruppen aufweisen, so liegt die Reaktionstemperatur bevorzugt in einem Bereich von etwa 5 bis 80 °C, besonders bevorzugt 5 bis 40 °C. Die Reaktion kann dann gewünschtenfalls in einem Lösungsmittel oder Lösungsmittelgemisch erfolgen, welches aktive Wasserstoffatome aufweisen kann. Neben den zuvor genannten werden dann bevorzugt Alkohole, wie Methanol und Ethanol, Gemische aus Alkoholen und Wasser sowie Gemische aus Alkoholen und den zuvor genannten Ketonen eingesetzt. Weisen die resultierenden Polyurethane noch freie Isocyanatgruppen auf, so werden diese abschließend durch Zusatz von Aminen, vorzugsweise Aminoalkoholen, inaktiviert. Geeignete Aminoalkohole sind die zuvor beschriebenen, bevorzugt 2-Amino-2-methyl-1-propanol.

Die zur Herstellung der erfindungsgemäßen Polyurethane eingesetzten Oligomere a) können nach einer geeigneten Ausführungsform, wie zuvor beschrieben, separat hergestellt und vor ihrem Einsatz bei der Polyurethanherstellung nach üblichen Verfahren isoliert und/oder gereinigt werden.

Nach einer bevorzugten Ausführungsform erfolgt die Herstellung der Oligomere a) und die Herstellung der erfindungsgemäßen Polyurethane ohne Isolierung eines Zwischenprodukts. Vorzugsweise erfolgen beide Umsetzungen nacheinander in dem selben Reaktionsgefäß.

### Vorzugsweise enthalten die Polyurethane

- 0,3 bis 50 Gew.-%, bevorzugt 0,5 bis 40 Gew.-%, wenigstens eines Oligomers a),
- 0,5 bis 25 Gew.-%, bevorzugt 1 bis 20 Gew.-%, wenigstens einer Komponente b),
- 0,5 bis 50 Gew.-%, bevorzugt 3 bis 45 Gew.-%, wenigstens einer Komponente c),
- 0 bis 25 Gew.-%, bevorzugt 0,01 bis 15 Gew.-%, wenigstens einer Komponente d),
- 25 bis 60 Gew.-%, bevorzugt 35 bis 53 Gew.-%, wenigstens einer Komponente e)
einpolymerisiert.

Die Säuregruppen enthaltenden Polyurethane können mit einer Base teilweise oder vollständig neutralisiert werden. Die Amingruppen enthaltenden Polyurethane können teilweise oder vollständig protoniert oder quaternisiert werden.

In aller Regel weisen die erhaltenen Salze der Polyurethane eine bessere Wasserlöslichkeit oder Dispergierbarkeit in Wasser auf als die nicht neutralisierten Polyurethane. Als Base für die Neutralisation der Polyurethane können Alkalimetallbasen wie Natronlauge, Kalilauge, Soda, Natriumhydrogencarbonat, Kaliumcarbonat oder Kaliumhydrogencarbonat und Erdalkalimetallbasen wie Calciumhydroxyd, Calciumoxid, Magnesiumhydroxyd oder Magnesiumcarbonat sowie Ammoniak und Amine verwendet werden. Geeignete Amine sind z. B. C₁-C₆-Alkylamine, bevorzugt n-Propylamin und n-Butylamin, Dialkylamine, bevorzugt Diethylpropylamin und Dipropylmethylamin, Trialkylamine, bevorzugt Triethylamin und Triisopropylamin, C₁-C₆-Alkyldiethyanolamine, bevorzugt Methyl- oder Ethyldiethanolamin und Di-C₁-C₆-Alkylethanolamine sowie Glucamin und Methylglucamin. Besonders für den Einsatz in Haarbehandlungsmitteln haben sich zur Neutralisation der Säuregruppen enthaltenden Polyurethane 2-Amino-2-methyl-1-propanol, Diethylaminopropylamin und Triisopropanolamin bewährt. Die Neutralisation der Säuregruppen enthaltenden Polyurethane kann auch mit Hilfe von Mischungen mehrerer Basen vorgenommen werden, z. B. Mischungen aus Natronlauge und Triisopropanolamin. Die Neutralisation kann je nach Anwendungszweck partiell z. B. zu 20 bis 40 % oder vollständig, d. h. zu 100 % erfolgen.

Die Amingruppen bzw. protonierte oder quaternisierte Amingruppen enthaltenden Polyurethane sind aufgrund ihrer kationischen Gruppen im Allgemeinen leicht in Wasser oder Wasser/Alkohol-Gemischen löslich oder zumindest ohne Zuhilfenahme von Emulgatoren dispergierbar. Geladene kationische Gruppen lassen sich aus den vorliegenden tertiären Aminstickstoffen entweder durch Protonierung, z. B. mit Carbonsäuren, wie Milchsäure, oder Mineralsäuren, wie Phosphorsäure, Schwefelsäure und Salzsäure, oder durch Quaternisierung, z. B. mit Alkylierungsmitteln, wie C₁- bis C₄-Alkylhalogeniden oder -sulfaten, erzeugen. Beispiele solcher Alkylierungsmittel sind Ethylchlorid, Ethylbromid, Methylchlorid, Methylbromid, Dimethylsulfat und Diethylsulfat.

Polyurethane, die sowohl kationogene als auch anionogene Gruppen aufweisen, können nacheinander einer Neutralisation mit wenigstens einer Säure, einer Neutralisation mit wenigstens einer Base und gewünschtenfalls zusätzlich einer Quaternisierung unterzogen werden. Die Reihenfolge der Neutralisationsschritte ist dabei im Allgemeinen beliebig.

Wird bei der Herstellung der polymeren Salze ein wassermischbares organisches Lösungsmittel eingesetzt, so kann dieses im Anschluss durch übliche, dem Fachmann bekannte Verfahren, z. B. durch Destillation bei vermindertem Druck, entfernt werden. Vor dem Abtrennen des Lösungsmittels kann dem polymeren Salz zusätzlich Wasser zugegeben werden. Nach Ersatz des Lösungsmittels durch Wasser erhält man eine Lösung oder Dispersion des polymeren Salzes, aus der, falls gewünscht, das polymere Salz in üblicher Weise gewonnen werden kann, z. B. durch Sprühtrocknung.

Der pH-Wert der wässrigen Lösungen oder Dispersionen kann durch Zugabe einer Säure oder Base eingestellt werden. Geeignete Säuren und Basen sind die zuvor als zusätzliche Neutralisierungsmittel genannten. Vorzugsweise liegt der pH-Wert für anionische Polyurethane im alkalischen Bereich, insbesondere > 7,5. Vorzugsweise liegt der pH-Wert für kationische Polyurethane im sauren Bereich, insbesondere bei 5,5 bis 6,5.

Die erfindungsgemäßen Polyurethane sind wasserlöslich oder wasserdispergierbar. Sie bilden im Allgemeinen klare und klebfreie Filme und lassen sich mit Wasser sehr gut auswaschen. Vorteilhafterweise werden mit den erfindungsgemäßen Polyurethane auch Filme mit einer sehr guten Elastizität erhalten. Diese ist im Allgemeinen höher als die Elastizität, die üblicherweise bei aus dem Stand der Technik bekannten Polyurethanen erhalten wird. Haarbehandlungsmittel auf Basis dieser Polymere verleihen dem Haar eine sehr gute Geschmeidigkeit.

Die erfindungsgemäßen Polyurethane bzw. Polyharnstoffe sind im Allgemeinen ohne Zuhilfenahme von Emulgatoren in Alkoholen, Alkohol/Wasser-Gemischen und/oder in Wasser löslich bzw. dispergierbar. Bevorzugte Alkohole zur Herstellung von Formulierungen der erfindungsgemäßen Polyurethane sind insbesondere C₁- bis C₄-Alkanole, wie Methanol, Ethanol, n-Propanol, Isopropanol, n-Butanol und Mischungen davon.

Die erfindungsgemäßen Polyurethane sind als Hilfsmittel in der Kosmetik, bevorzugt als oder in Beschichtungsmittel(n) für keratinhaltige und keratinanaloge Oberflächen, wie Haar, Haut und Nägel, brauchbar. Sie eignen sich insbesondere für die Haarkosmetik, vorzugsweise als Festigerpolymer in Haarsprays, Schaumfestigern, Haarmousse, Haargel und Shampoos. Sie eignen sich weiterhin bevorzugt für eine Verwendung in der dekorativen Kosmetik, insbesondere in Mascara, Make-up und Lidschatten. Sie können weiterhin vorteilhaft als polymerer Emulgator oder Coemulgator zur Formulierung von kosmetischen oder pharmazeutischen Präparaten für die Haut eingesetzt werden. Aufgrund ihrer hydrophilen wie hydrophoben Eigenschaften eignen sie sich sowohl für öl- und fetthaltige Zubereitungen, wie auch für O/W- und W/O-Emulsionen. Die erfindungsgemäßen Polyurethane sind weiterhin als Hilfsmittel in der Pharmazie, bevorzugt als oder in Beschichtungsmittel(n) oder Bindemittel(n) für feste Arzneiformen brauchbar. Die zuvor genannten Polyurethane können auch in Cremes und als Tablettenüberzugmittel und Tablettenbindemittel verwendet werden. Sie eignen sich auch als Bindemittel und Klebemittel für kosmetische Produkte. Die erfindungsgemäßen Polyurethane eignen sich weiterhin vorzugsweise für die Verwendung als oder in Beschichtungsmittel(n) für die Textil-, Papier-, Druck-, Leder- und Klebstoffindustrie.

Ein weitere Gegenstand der Erfindung ist ein kosmetisches oder pharmazeutisches Mittel, das wenigstens ein erfindungsgemäßes Polyurethan enthält. Im Allgemeinen enthält das Mittel die Polyurethane in einer Menge im Bereich von etwa 0,2 bis 30 Gew.-%, bevorzugt 0,5 bis 20 Gew.-%, bezogen auf das Gesamtgewicht des Mittels.

Die erfindungsgemäßen kosmetischen Mittel eignen sich insbesondere als Beschichtungsmittel für keratinhaltige und keratinanaloge Oberflächen (Haar, Haut und Nägel). Die in ihnen eingesetzten Verbindungen sind wasserlöslich oder wasserdispergierbar. Sind die in den erfindungsgemäßen Mitteln eingesetzten Verbindungen wasserdispergierbar, können sie in Form von wässrigen Mikrodispersionen mit Teilchendurchmessern von üblicherweise 1 bis 350 nm, bevorzugt 1 bis 250 nm, zur Anwendung gebracht werden. Die Feststoffgehalte der Präparate liegen dabei üblicherweise in einem Bereich von etwa 0,5 bis 20 Gew.-%, bevorzugt 1 bis 12 Gew.-%. Diese Mikrodispersionen benötigen in der Regel keine Emulgatoren oder Tenside zu ihrer Stabilisierung.

Bevorzugt können die erfindungsgemäßen Mittel in Form eines Haarbehandlungsmittels, wie Schaumfestiger, Haarmousse, Haargel, Shampoo und insbesondere in Form eines Haarsprays vorliegen. Zur Anwendung als Haarfestiger sind dabei Mittel bevorzugt, die Polyurethane enthalten, die wenigstens eine Glasübergangstemperatur T_{g} ≥ 10 °C, bevorzugt ≥ 20 °C, aufweisen. Der K-Wert dieser Polymere (gemessen nach E. Fikentscher, Cellulose-Chemie 13 (1932), S. 58-64) an einer 1 gew.-%igen Lösung in N-Methylpyrrolidon, liegt vorzugsweise in einem Bereich von 23 bis 90, insbesondere 25 bis 60. weisen die erfindungsgemäßen Polyurethane Siloxangruppen auf, so beträgt der Siloxangehalt dieser Polymere im Allgemeinen 0,05 bis 20 Gew.-%, bezogen auf das Gesamtgewicht der eingebauten Komponenten.

Vorzugsweise handelt es sich um Haarbehandlungsmittel. Diese liegen üblicherweise in Form einer wässrigen Dispersion oder in Form einer alkoholischen oder wässrig-alkoholischen Lösung vor. Beispiele geeigneter Alkohole sind Ethanol, Propanol, Isopropanol etc.

Weiter können die erfindungsgemäßen Haarbehandlungsmittel im Allgemeinen übliche kosmetische Hilfsstoffe enthalten, beispielsweise Weichmacher, wie Glycerin und Glykol; Emollienzien; Parfüms; Tenside; UV-Absorber; Farbstoffe; antistatische Mittel; Mittel zur Verbesserung der Kämmbarkeit; Konservierungsmittel; und Entschäumer.

Wenn die erfindungsgemäßen Mittel als Haarspray formuliert sind, enthalten sie eine ausreichende Menge eines Treibmittels, beispielsweise einen niedrigsiedenden Kohlenwasserstoff oder Ether, wie Propan, Butan, Isobutan oder Dimethylether. Als Treibmittel sind auch komprimierte Gase brauchbar, wie Stickstoff, Luft oder Kohlendioxid. Die Menge an Treibmittel kann dabei gering gehalten werden, um den VOC-Gehalt nicht unnötig zu erhöhen. Sie beträgt dann im Allgemeinen nicht mehr als 55 Gew.-%, bezogen auf das Gesamtgewicht des Mittels. Gewünschtenfalls sind aber auch höhere VOC-Gehalte von 85 Gew.-% und darüber möglich.

Die zuvor beschriebenen Polyurethane können auch in Kombination mit anderen Haarpolymeren in den Mitteln zur Anwendung kommen. Solche Polymere sind insbesondere:
- nicht-ionische, wasserlösliche bzw. wasserdispergierbare Polymere oder Oligomere, wie Polyvinylcaprolactam, z. B. Luviskol Plus (BASF), oder Polyvinylpyrrolidon und deren Copolymere, insbesondere mit Vinylestern, wie Vinylacetat, z. B. Luviskol VA 37 (BASF); Polyamide, z. B. auf Basis von Itaconsäure und aliphatischen Diaminen, wie sie z. B. in der DE-A-43 33 238 beschrieben sind; Polyvinylalkohole und Derivate davon; Polymere auf Cellulosebasis;
- amphotere oder zwitterionische Polymere, wie die unter den Bezeichnungen Amphomer® (National Starch) erhältlichen Octylacrylamid/Methylmethacrylat/tert.-Butylaminoethylmethacrylat/2-Hydroxypropylmethacrylat-Copolymere sowie zwitterionische Polymere, wie sie beispielsweise in den deutschen Patentanmeldungen DE 39 29 973, DE 21 50 557, DE 28 17 369 und DE 37 08 451 offenbart sind. Acrylamidopropyltrimethylammoniumchlorid/Acrylsäure- bzw. -Methacrylsäure-Copolymerisate und deren Alkali- und Ammoniumsalze sind bevorzugte zwitterionische Polymere. Weiterhin geeignete zwitterionische Polymere sind Methacroylethylbetain/Methacrylat-Copolymere, die unter der Bezeichnung Amersette® (AMERCHOL) im Handel erhältlich sind, und Copolymere aus Hydroxyethylmethacrylat, Methylmethacrylat, N,N-Dimethylaminoethylmethacrylat und Acrylsäure (Jordapon®);
- anionische Polymere, wie Vinylacetat/Crotonsäure-Copolymere, wie sie beispielsweise unter den Bezeichnungen Resyn® (NATIONAL STARCH), Luviset® (BASF) und Gafset® (GAF) im Handel sind, vinylpyrrolidon/Vinylacrylat-Copolymere, erhältlich beispielsweise unter dem Warenzeichen Luviflex® (BASF). Ein bevorzugtes Polymer ist das unter der Bezeichnung Luviflex® VBM-35 (BASF) erhältliche Vinylpyrrolidon/Acrylat-Terpolymer. Acrylsäure/Ethylacrylat/N-tert.Butylacrylamid-Terpolymere, die beispielsweise unter der Bezeichnung Ultrahold® strong (BASF) vertrieben werden, sowie Luvimer® (BASF, Terpolymer aus t-Butylacrylat, Ethylacrylat und Methacrylsäure), Natriumsulfonat-haltige Polyamide oder Natriumsulfonat-haltige Polyester, oder
- kationische (quaternisierte) Polymere, z. B. kationische Polyacrylatcopolymere auf Basis von N-Vinyllactamen und deren Derivaten (N-Vinylpyrrolidon, N-Vinylcaprolactam etc.) sowie übliche kationische Haarconditionerpolymere, z. B. Luviquat® (Copolymer aus Vinylpyrrolidon und Vinylimidazoliummethochlorid), Luviquat® Hold (Copolymerisat aus quaternisiertem N-Vinylimidazol, N-Vinylpyrrolidon und N-Vinylcaprolactam), Merquat® (Polymer auf Basis von Dimethyldiallylammoniumchlorid), Gafquat® (quaternäre Polymere, die durch Reaktion von Polyvinylpyrrolidon mit quaternären Ammoniumverbindungen entstehen), Polymer JR (Hydroxyethylcellulose mit kationischen Gruppen), Polyquaternium-Typen (CTFA-Bezeichnungen) etc., Chitosan und Chitosanderivate;
- nichtionische, siloxanhaltige, wasserlösliche oder -dispergierbare Polymere, z. B. Polyethersiloxane, wie Tegopren® (Fa. Goldschmidt) oder Belsil® (Fa. Wacker).

Die erfindungsgemäßen Oligomer können als Mischung mit einem amidgruppenhaltigen Haarpolymer eingesetzt. Dazu zählen z. B. die in der DE-A-42 25 045 beschriebenen Polyurethane, die zuvor beschriebenen vinylpyrrolidon/Acrylat-Terpolymere und Acrylsäure/Ethylacrylat/N-tert.-Butylacrylamid-Terpolymere (z. B. Ultrahold®strong der BASF AG), die zuvor beschriebenen amidgruppenhaltigen amphoteren Polymere (z. B. Amphomer®) und insbesondere Copolymerisate, die einen Anteil an amidgruppenhaltigen Monomeren, wie N-Vinyllactamen, von mindestens 30 Gew.-% aufweisen (z. B. Luviskol®plus und Luviskol®VA37 der BASF AG).

Die anderen Haarpolymere sind vorzugsweise in Mengen bis zu 10 Gew.-%, bezogen auf das Gesamtgewicht des Mittels enthalten.

Ein bevorzugtes Haarbehandlungsmittel enthält:
a) 0,5 bis 20 Gew.-%, bevorzugt 1 bis 10 Gew.-%, mindestens eines in Wasser löslichen oder dispergierbaren, erfindungsgemäßen Polyurethans,
b) 50 bis 99,5 Gew.-%, bevorzugt 55 bis 99 Gew.-%, eines Lösungsmittels, ausgewählt unter Wasser und wassermischbares Lösungsmitteln, bevorzugt C₂- bis C₅-Alkoholen, insbesondere Ethanol, und Mischungen davon,
c) 0 bis 70 Gew.-%, bevorzugt 0,1 bis 50 Gew.-%, eines Treibmittels, vorzugsweise ausgewählt unter Dimethylether und Alkanen, wie z. B. Propan/Butan-Gemischen,
d) 0 bis 10 Gew.-%, bevorzugt 0,1 bis 10 Gew.-%, mindestens eines von a) verschiedenen, in Wasser löslichen oder dispergierbaren Haarpolymers,
e) 0 bis 0,5 Gew.-%, bevorzugt 0,001 bis 2 Gew.-%, mindestens einer wasserlöslichen oder wasserdispergierbaren Siliconverbindung,
f) 0 bis 1 Gew.-%, bevorzugt 0,0001 bis 0,5 Gew.-%, wenigstens einer Verbindung, die ausgewählt ist unter Estern und Amiden von gesättigten und einfach oder mehrfach ungesättigten C₅₋bis C₃₀-Carbonsäuren, gesättigten und einfach oder mehrfach ungesättigten C₈- bis C₃₀-Alkoholen und Mischungen davon.
sowie übliche Zusatzstoffe.

Das erfindungsgemäße Mittel kann als Komponente d) mindestens ein anderes, in Wasser lösliches oder dispergierbares Haarpolymer enthalten. Der Anteil dieser Komponente beträgt dann im Allgemeinen etwa 0,1 bis 10 Gew.-%, bezogen auf das Gesamtgewicht des Mittels. Bevorzugt können dabei wasserlösliche oder wasserdispergierbare Polyurethane eingesetzt werden, die keine Siloxangruppen einpolymerisiert enthalten.

Das erfindungsgemäße Mittel kann als Komponente e) mindestens ein nichtionisches, siloxanhaltiges, wesserlösliches oder -dispergierbares Polymer, insbesondere ausgewählt unter den zuvor beschriebenen Polyethersiloxanen, enthalten. Der Anteil dieser Komponente beträgt dann im Allgemeinen etwa 0,001 bis 2 Gew.-%, bezogen auf das Gesamtgewicht des Mittels.

Das erfindungsgemäße Mittel kann als zusätzliche Komponente mindestens ein wasserunlösliches Silicon, insbesondere ein Polydimethylsiloxan, z. B. die Abil®-Typen der Fa. Goldschmidt, enthalten. Der Anteil dieser Komponente beträgt dann im Allgemeinen etwa 0,0001 bis 0,2 Gew.-%, bevorzugt 0,001 bis 0,1 Gew.-%, bezogen auf das Gesamtgewicht des Mittels.

Das erfindungsgemäße Mittel kann zusätzlich gegebenenfalls einen Entschäumer, z. B. auf Silicon-Basis, enthalten. Die Menge des Entschäumers beträgt im Allgemeinen bis zu etwa 0,001 Gew.-%, bezogen auf die Gesamtmenge des Mittels.

Bevorzugt enthält das erfindungsgemäße Mittel als Komponente f) wenigstens eine Ester der allgemeinen Formel R²⁰-CO-OR²¹, worin R²⁰ für einen geradkettigen oder verzweigten C₅- bis C₃₀-, bevorzugt C₁₀- bis C₂₀-Alkyl- oder -Alkenylrest steht, wobei der Alkenylrest 1,2,3 oder 4 nicht benachbarte Doppelbindungen aufweisen kann. R²¹ steht vorzugsweise für einen geradkettigen oder verzweigten C₁₋bis C₃₀-, insbesondere C₅- bis C₂₂-Alkylrest oder für einen geradkettigen oder verzweigten C₆- bis C₃₀-, insbesondere C₈- bis C₂₂-Alkenylrest, der 1,2,3 oder 4 nicht benachbarte Doppelbindungen aufweisen kann.

Bevorzugt werden die Ester der Capronsäure, Carpylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren Mischungen, mit Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Eurcylalkohol und Brassidyalkohol sowie deren Mischungen, eingesetzt. Vorzugsweise werden Ester eingesetzt, die in Fettsäure und Fettalkoholkomponente zusammen mindestens 24, vorzugsweise mindestens 30 Kohlenstoffatome und gegebenenfalls eine Doppelbindung aufweisen. Typische Beispiele sind Oleylerucat, Erucyloleat, Behenyloleat und Ceteraryloleat.

Weiterhin sind als Komponenten f) Amide der allgemeinen Formel R²⁰-CO-NR²²R²³ bevorzugt, wobei R²⁰ die zuvor angegebenen Bedeutungen aufweisen kann und R²² und R²³ unabhängig voneinander für Wasserstoff, C₁- bis C₁₂-Alkyl oder C₅- bis C₈-Cycloalkyl stehen.

Weiterhin sind als Komponente f) gesättigte sowie einfach oder mehrfach ungesättigte Alkohole bevorzugt. Geeignete C₈- bis C₃₀-Alkylreste sind die zuvor genannten. Die Alkohole können einzeln oder als Gemische eingesetzt werden. Solche Alkohole und Alkoholgemische sind z. B. erhältlich durch Hydrierung von Fettsäuren aus natürlichen Fetten und Ölen oder von synthetischen Fettsäuren, z. B. aus der katalytischen Oxidation von Paraffinen. Geeignete Alkohole und Alkoholgemische sind weiterhin erhältlich durch Hydroformylierung von Olefinen mit gleichzeitiger Hydrierung der Aldehyde, wobei im Allgemeinen Gemische aus geradkettigen und verzweigten primären Alkoholen (Oxo-Alkohole) resultieren. Geeignete Alkohole und Alkoholgemische sind weiterhin erhältlich durch partielle Oxidation von n-Paraffinen nach bekannten Verfahren, wobei überwiegend lineare sekundäre Alkohole erhalten werden. Geeignet sind weiterhin die im wesentlichen primären, geradkettigen und geradzahligen Ziegler-Alkohole.

Bevorzugte Alkohole f) sind die sogenannten Guerbetalkohole. Guerbetalkohole, die bevorzugt als Komponente f) eingesetzt werden, werden vorzugsweise durch basenkatalysierte Eigenkondensation von linearen und/oder verzweigten Alkoholen mit 6 bis 22 und vorzugsweise 8 bis 18 Kohlenstoffatomen erhalten. Eine Übersicht hierzu findet sich in A. J. O'Lennick in Soap Cosm. Chem. Spec. (April) 52 (1987). Typische Beispiele sind Kondensationsprodukte von technischen Fettalkoholschnitten mit 8 bis 10 bzw. 16 bis 18 Kohlenstoffatomen.

Die zuvor als Komponente f) genannten Verbindungen können einzeln oder als Mischungen eingesetzt werden. Die erfindungsgemäßen kosmetischen Mittel, die die Komponente f) enthalten, weisen im Allgemeinen auch ohne einen Einsatz von Silicon-haltigen Komponenten gute anwendungstechnische Eigenschaften, wie z. B. eine gute Flexibilität und einen angenehmen Griff auf.

Das erfindungsgemäße Mittel enthält vorzugsweise zusätzlich zu den zuvor genannten Komponenten:
g) 0 bis 40 Gew.-%, bevorzugt 0,1 bis 35 Gew.-%, wenigstens eines Tensids,
h) 0 bis 5 Gew.-%, bevorzugt 0,05 bis 4 Gew.-%, wenigstens eines Farbstoffes und/oder UV-Absorbers,
i) 0 bis 3 Gew.-%, bevorzugt 0,05 bis 2,5 Gew.-%, wenigstens eines Salzes,
k) 0 bis 3 Gew.-%, bevorzugt 0,05 bis 2,5 Gew.-%, wenigstens eines Verdickers,
sowie gegebenenfalls weitere übliche Zusatzstoffe. Diese liegen dann im Allgemeinen jeweils in einer Menge von etwa 0 bis 0,2 Gew.-%, bevorzugt 0,001 bis 0,2 Gew.-%, bezogen auf das Gesamtgewicht des Mittels, vor.

Die erfindungsgemäßen Mittel besitzen den Vorteil, dass sie einerseits den Haaren die gewünschte Festigkeit verleihen und die Polymere leicht auswaschbar (redispergierbar) sind und das Haar andererseits elastisch bleibt.

Vorteilhafterweise eignen sich die erfindungsgemäßen Polyurethane auch als Komponente in Haarbehandlungsmitteln, die zusätzlich wenigstens ein weiteres herkömmliches Haarpolymer enthalten. Auch mit diesen Mischungen werden im Allgemeinen bessere Flexibilitäten erzielt als bei den entsprechenden Polymeren oder Mischungen, die die erfindungsgemäßen Polyurethane nicht enthalten. Die erfindungsgemäßen Polyurethane eigenen sich somit auch zur Verbesserung der Elastizität herkömmlicher Haarfestigungsmittel. Diese verleihen den Haaren dann im Allgemeinen eine sehr gute Flexibilität und Geschmeidigkeit.

Die Erfindung wird anhand der folgenden, nicht einschränkenden Beispiele näher erläutert.

### I. Herstellung von Oligomeren

### Oligomer O1:

### Herstellung eines Polyurethandiols aus Hexamethylendiisocyanat und Neopentylglykol

In einem 4-Halskolben, der mit Rührer, Tropftrichter, Thermometer, Rückflusskühler und einer Vorrichtung für das Arbeiten unter Stickstoff ausgestattet war, wurden 624 g (6 mol) Neopentylglykol und 0,3 Tetrabutylorthotitanat in 490 g Methylethylketon unter Erhitzen auf eine Temperatur von etwa 50 °C und unter Rühren gelöst. Anschließend wurden unter Rühren 840 g (5 mol) Hexamethylendiisocyanat zugetropft, wobei die Reaktionstemperatur anstiegt. Unter Rückfluss wurde das Reaktionsgemisch dann zwei Stunden gerührt und anschließend unter Rühren auf Raumtemperatur abgekühlt. Man erhielt eine klare, hochviskose 75 gew.-%ige Lösung eines Polyurethandiols mit einer OH-Zahl von etwa 75 und einem zahlenmittleren Molekulargewicht von etwa 1500 g/mol.

### Oligomer O2:

### Herstellung eines Polyurethans auf Basis von Polytetrahydrofuran

In einem 4-Halskolben, der mit Rührer, Tropftrichter, Thermometer, Rückflusskühler und einer Vorrichtung für das Arbeiten unter Stickstoff ausgestattet war, wurden 650 g (1 mol) Polytetrahydrofuran (Mₙ = 650 g/mol) und 0,1 g Tetrabutylorthotitanat vorgelegt und die Mischung unter Rühren auf etwa 50 °C erwärmt. Anschließend wurden unter Rühren 109,5 g (0,65 mol) Hexamethylendiisocyanat zugetropft, wobei die Reaktionstemperatur anstieg. Das Reaktionsgemisch wurde weitere drei Stunden bei 80 °C gerührt. Nach dem Abkühlen auf Raumtemperatur erhielt man ein wachsartiges Produkt mit eine OH-Zahl von etwa 50 mit einem zahlenmittleren Molekulargewicht von etwa 2250 g/mol.

Die Herstellung des Oligomers 02 kann auch analog der Herstellungsvorschrift für das Oligomer Ol, d. h. in einem Lösungsmittel, wie Methylethylketon, erfolgen.

### Oligomer O3:

### Herstellung eines Polyurethans auf Basis eines Polyesterdiols

Durch Polykondensation von 3,5 mol Sebacinsäure und 4,5 mol 1,6-Hexandiol wurde ein Hydroxylgruppen-haltiges Polyesterdiol mit einem zahlenmittleren Molekulargewicht von etwa 1 150 g/mol erhalten. Analog der Herstellungsvorschrift für das Oligomer 02 wurden 1 mol des Polyesterdiols und 0,65 mol Hexamethylendiisocyanat umgesetzt. Man erhielt ein hartes wachsartiges Produkt mit einer OH-Zahl von etwa 30 und einem zahlenmittleren Molekulargewicht von etwa 3700 g/mol.

Analog zu Oligomer 03 wurde das Oligomer 04 hergestellt. Die eingesetzten Edukte und deren Mengen finden sich in der folgenden Tabelle 1.

### Oligomer 05:

### Herstellung eines Polyurethans auf Basis eines Polysiloxandiols

Nach der allgemeinen Herstellungsvorschrift für das Oligomer O1 wurden 1 mol Polysiloxandiol (Mₙ = 900 g/mol, Tegomer® H-Si 2122 der Fa. Goldschmidt) und 0,65 mol Hexamethylendiisocyanat in Methylethylketon umgesetzt.

Die Herstellung von Oligomeren dieses Typs kann auch nach der allgemeinen Vorschrift für das Oligomer 02, d. h. ohne Zusatz eines Lösungsmittels, erfolgen.

### Oligomer 06:

### Herstellung eines Polyurethans auf Basis eines Polysiloxandiamins

Analog der Herstellungsvorschrift für das Oligomer 01 wurden 1 mol Polysiloxandiamin (Mₙ = 900 g/mol, Tegomer® A-Si 2122 der Fa. Goldschmidt) und 0,65 mol Hexamethylendiisocyanat in Methylethylketon bei einer Temperatur von etwa 30°C und ohne Zusatz eines Katalysators umgesetzt.

### Oligomer O7:

### Herstellung eines Hydroxylgruppen-haltigen Reaktionsproduktes eines Polyurethandiols mit einer Dimerfettsäure

In einem 4-Halskolben, der mit Rührer, Tropftrichter, Thermometer, Rückflusskühler und einer Vorrichtung für das Arbeiten unter Stickstoff ausgestattet war, wurde nach der allgemeinen Herstellungsvorschrift für das Oligomer O1 ein Polyurethandiol aus 2 mol 1,6-Hexandiol und 1 mol Hexamethylendiisocyanat hergestellt. Anschließend wurde der Rückflusskühler durch einen absteigenden Kühler ersetzt. Unter Stickstoffatmosphäre wurden 0,65 mol einer Dimerfettsäure zugegeben und das Reaktionsgemisch erwärmt, wobei zunächst das Methylethylketon bei einer Temperatur von 80 bis 110 °C abdestilliert wurde. Nach weiterer Temperaturerhöhung auf 160 °C wurde das Reaktionsgemisch weitere drei Stunden gerührt und die Reaktionstemperatur dann stündlich um etwa 20 °C gesteigert, bis sie etwa 210 °C erreicht hatte. Nach weiteren fünf Stunden bei etwa 210 °C lag die Säurezahl unter 2. Man ließ auf Raumtemperatur abkühlen und erhielt ein gelbes, fast klares Wachs mit einer OH-Zahl von etwa 48 und einem zahlenmittleren Molekulargewicht von etwa 2300 g/mol.

### Oligomer 08:

### Herstellung eines Hydroxylgruppen-haltigen Reaktionsproduktes eines Polyurethans mit Sebacinsäure

Analog der allgemeinen Herstellungsvorschrift für das Reaktionsprodukt 07 wurde zunächst ein Polyurethandiol aus 2 mol 1,6-Hexandiol und 1 mol Hexamethylendiisocyanat hergestellt und anschließend mit 0,65 mol Sebacinsäure umgesetzt.

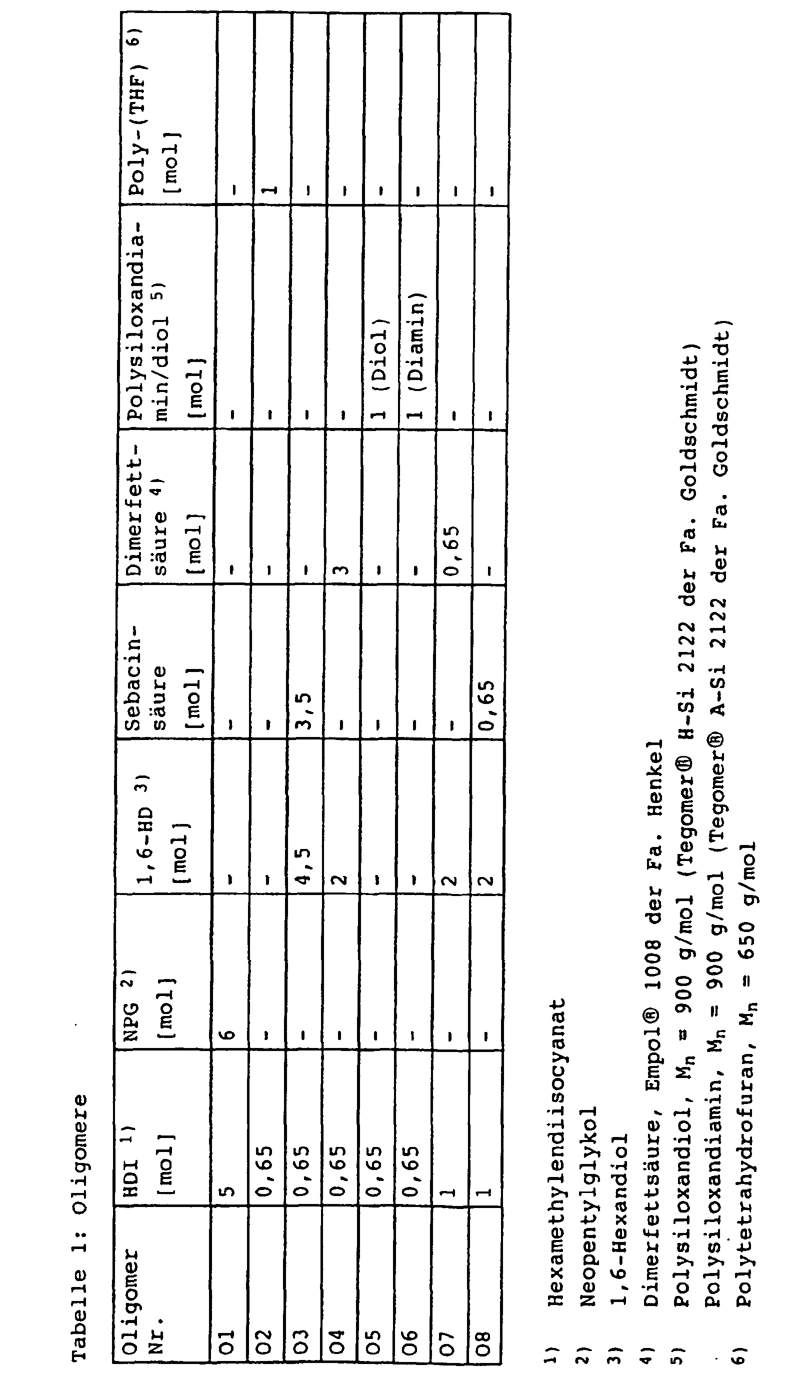

### II. Herstellung von Polymeren (nicht erfindungsgemäß)

### II.1: Öllösliche Polymere P9 bis P11

Analog der Herstellungsvorschrift zu Oligomer 03 wurden die Polymere P9 bis P11 aus Edukten gemäß Tabelle 2 hergestellt.

### II.2: Wasserlösliche Polymere P12 und P13

Analog der Herstellungsvorschrift zu Oligomer 02 wurden die Polymere P12 und P13 aus Edukten gemäß der Tabelle 2 in Masse hergestellt.

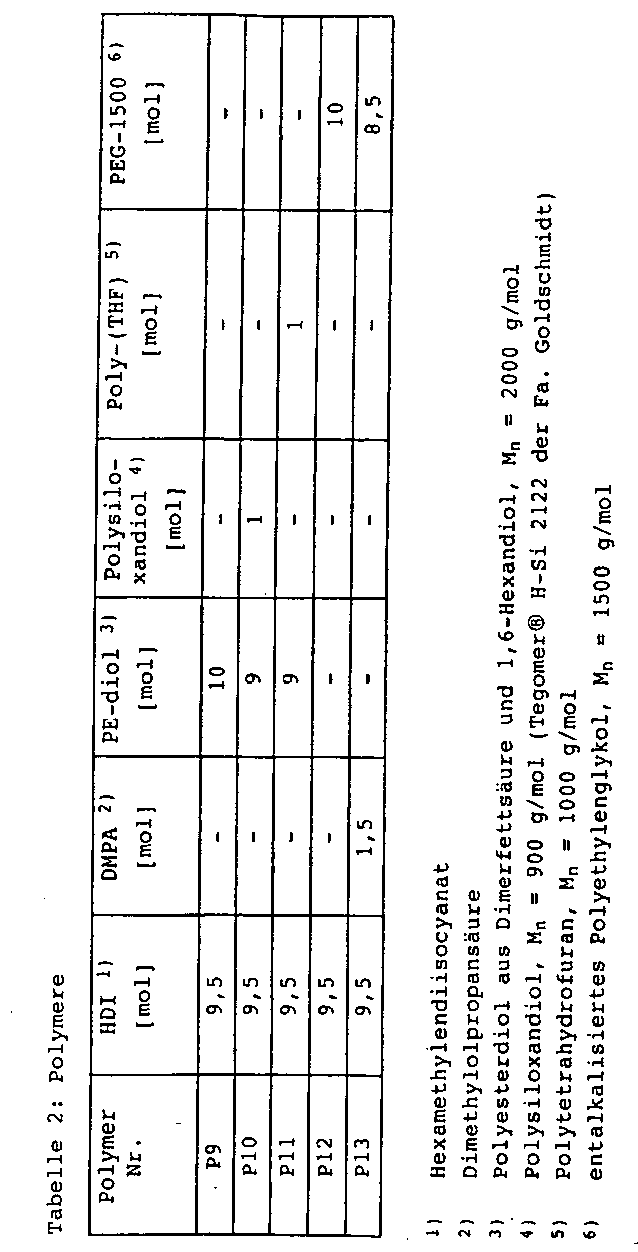

Anwendungstechnische Beispiele (Oligomere 01 bis 08 und öllösliche Polymere P9 bis P11):

Zur Herstellung von Formulierungen der zuvor genannten Oligomere und Polymere werden diese mit einem C₁₂- bis C₁₅-Alkylbenzoat (Finsolv® TN) in einer Menge gemäß Tabelle 3 gemischt und die Eigenschaften der resultierenden Mischungen beurteilt.

**Tabelle 3:**

| Beispiel Nr. | Ölkomponente | | |
|---|---|---|---|
| | 20 Gew.-% | 10 Gew.-% | 5 Gew.-% |
| 01 | unlöslich | unlöslich | unlöslich |
| 02 | fast klares viskoses Öl | fast klares viskoses Öl | klares viskoses Öl |
| 03 | festes, leicht trübes Wachs | festes, fast klares Wachs | gerade festes, fast klares Wachs |
| 04 | festes klares Wachs | festes, klares Wachs | klares hochviskoses Öl |
| 05 | fast klares, viskoses Öl, trennt sich in zwei Phasen beim Stehen | fast klares, viskoses Öl, trennt sich in zwei Phasen beim Stehen | fast klares, viskoses Öl, trennt sich in zwei Phasen beim Stehen |
| 06 | trübes viskoses Öl, trennt sich in zwei Phasen beim Stehen | leicht trübes viskoses Öl, trennt sich in zwei Phasen beim Stehen | fast klares, viskoses Öl, trennt sich in zwei Phasen beim Stehen |
| 07 | sehr festes, klares Wachs | festes, klares Wachs | klares hochviskoses Öl |
| 08 | sehr festes, klares Wachs | festes, leicht trübes Wachs | fast klares hochviskoses Öl |
| P9 | sehr festes, klares Wachs | festes, klares Wachs | gerade festes, klares Wachs |
| P10 | gerade festes, klares Wachs | gerade festes, klares Wachs; Veröligen beim Schütteln | klares viskoses Öl |
| P11 | festes, fast klares Wachs | gerade festes, fast klares Wachs; Veröligen beim Schütteln | klares viskoses Öl |

Formulierungsbeispiele für eine Verwendung in der Hautkosmetik (Oligomere O1 bis 08 und öllösliche Polymere P9 bis P11):

**Tabelle 4:**

| Einsatzstoff | Einsatzgebiet | | | |
|---|---|---|---|---|
| | Ölgrundlage | Salbengrundlage | Wachsgrundlage | Stiftgrundlage |
| Oligomer/Polymer aus Bsp.-Nr./ [Gew.-%] | 04, 07, 08, P9, P10, P11/ 5 | 04, 07, 08, P9/ 5 | 04, 07, P9/ 10 | 04, 07, 08, P9/ 30 |
| Paraffinöl [Gew.-%] | 10 | - | - | - |
| Kosmetisches Öl: | | | | |
| Finsolv® TN¹) | 60 | Fins . u/o | 65 | 60 |
| Miglyol ²⁾ | - | Migl. 30 | - | - |
| Ricinusöl [Gew.-%] | - | - | 15 | 10 |
| Paraffinwachs ³⁾ | - | 5 | 5 | - |
| Vaseline | 20 | 50 | 5 | - |
| Bienenwachs ⁴⁾ | 5 | 5 | - | - |
| Cetylalkohol | - | 5 | - | - |

| | | | | |
|---|---|---|---|---|
| ¹⁾ C₁₂-C₁₅-Alkylbenzoat | | | | |
| ²⁾ Neutralöl, Triglycerid gesättigter Pflanzenfettsäuren | | | | |
| ³⁾ Homopolymeres Polyethylenwachs (Luwax® A, BASF), Copolymeres Polyethylenwachs, Copolymer Acrylsäure (Luwax® EAS, BASF), Copolymeres Polyethylenwachs, Copolymer Vinylacetat (Luwax® EVA, BASF) und Gemische davon | | | | |
| 4) z. B. Berry Wax und/oder Ultrabee WD (beides Fa. Kahl, Deutschland) | | | | |

Die Formulierungen können weitere Komponenten, ausgewählt unter Farbpigmenten, öllöslichen Vitaminen, öllöslichen UV-Absorbern, Parfüm, etherischen Ölen, Konservierungsmitteln, etc. enthalten.

Beispiele für emulgatorfreie Emulsionen (Oligomere und öllösliche Polymere):

| Ölphase: | Gew.-% | Chemische Bezeichnung/ CTFA-Name: |
|---|---|---|
| Finsolv® TN | 18 | C₁₂-C₁₅-Alkylbenzoat |
| Oligomer/Polymer 04, 07, 08, P9, P10, P11 | 2 | |
| Nip Nip®, Nipa Laboratories Ltd. USA | 0,1 | Methyl- und Propyl-4-hydroxybenzoat (7:3) |

| Wasserphase: | Gew.-% | |
|---|---|---|
| Wasser | 79,2 | |
| Carbopol 940 | 0,4 | Polyacrylsäure (Fa. Goodrich) |
| Mowiol 4/88 | 0,2 | Polyvinylalkohol (Verseifungsgrad 88 %, Fa. Hoesch) |
| Germall 115 | 0,1 | Imidazolidinylharnstoff |

### Herstellung der emulgatorfreien Emulsionen

Zur Herstellung der emulgatorfreien Emulsionen werden die Komponenten für Öl- und Wasserphase getrennt eingewogen und die Ölphase bei ca. 100 °C und die Wasserphase bei ca. 60 °C homogenisiert. Dann gibt man die temperierte Ölphase (20 - 80 °C) langsam unter Rühren zu der ebenfalls gegebenenfalls temperierten Wasserphase (0 - 80 °C). Je höher die Temperaturen der Wasser- bzw. Ölphase sind, desto feiner sind die erhaltenen Emulsionen. Nach Neutralisation mit 2-Amino-2-methylpropanol (50 %ig in Wasser) auf pH 7,5 bis 8,5 werden stabile Emulsionen erhalten.

Beispiele für Cremes (Oligomere und öllösliche Polymere):

| Ölphase: | Gew.-% | Chemische Bezeichnung/ CTFA-Name: |
|---|---|---|
| Paraffinöl | 4,5 | |
| Cremophor® A6 (BASF AG) | 2,5 | Ceteareth-6 (Stearylalkohol-Ethoxylat) |
| Cremophor® A6 (BASF AG) | 2,5 | Ceteareth-25 (Fettalkohol-Ethoxylat) |
| Cetylalkohol | 3,5 | |
| Glycerinmonostearat s.e. | 2,5 | Glycerylstearat |
| Palmitinsäure-i-propylester | 2,0 | |
| Luvitol® EHO (BASF AG) | 6,2 | Ceteraryloctanoat |
| Oligomer/Polymer 04, 07, 08, P9, P10, P11 | 1,1 | |
| Nip Nip®, Nipa Laboratories Ltd. USA | 0,1 | Methyl- und Propyl-4-hydroxybenzoat (7:3) |

| Wasserphase: | Gew.-% | |
|---|---|---|
| Wasser | 75,0 | |
| Germall 115 | 0,1 | Imidazolidinylharnstoff |

| Ölphase: | Gew.-% | Chemische Bezeichnung/ CTFA-Name: |
|---|---|---|
| Paraffinöl | 4,5 | |
| Cremophor® A6 (BASF AG) | 2,5 | Ceteareth-6 (Stearylalkohol-Ethoxylat) |
| Cremophor® A6 (BASF AG) | 2,5 | Ceteareth-25 (Fettalkohol-Ethoxylat) |
| Luwax® A | 3,6 | |
| Glycerinmonostearat s.e. | 2,5 | Glycerylstearat |
| Palmitinsäure-i-propylester | 2,0 | |
| Luvitol® EHO (BASF AG) | 6,2 | Ceteraryloctanoat |
| Oligomer/Polymer 04, 07, 08, P9, P10, P11 | 1,0 | |
| Nip Nip®, Nipa Laboratories Ltd. USA | 0,1 | Methyl- und Propyl-4-hydroxybenzoat (7:3) |

| Wasserphase: | Gew.-% | |
|---|---|---|
| P12 oder P13 | 1,0 | |
| Wasser | 74,0 | |
| Germall 115 | 0,1 | Imidazolidinylharnstoff |

### Herstellung:

Einwiegen und unter Rühren die Ölphasen und Wasserphase getrennt bei einer Temperatur von ca. 80 °C homogenisieren. Wasserphase langsam in Ölphase einrühren. Unter Rühren langsam auf Raumtemperatur abkühlen.

Beispiele für die Verwendung in der Haarkosmetik (wasserlösliche Polymer 12 und 13):

**Gel mit wasserlöslichen Polymeren und Polyvinylalkohol**

| Zusammensetzung | Gew.-% |
|---|---|
| a) wässrige Lösung Carbopol 940 (1 %ig) ¹⁾ | 100 |
| b) wässrige Lösung Polymer 12 oder 13 (10 %ig) | 50 |
| c) wässrige Lösung Mowiol 4/88 (20 %ig) ²⁾ | 49 |
| d) AMP-Lösung (10 %ig) ³⁾ | 1 |

| | |
|---|---|
| 1) Carbopol 940: Polyacrylsäure (Fa. Goodrich) | |
| 2) Mowiol 4/88: Polyvinylalkohol (Verseifungsgrad 88 %, Hoesch) | |
| 3) AMP: Aminomethylpropanol | |

### Herstellung:

Ein Gemisch aus b), c) und d) wird unter Rühren auf eine Temperatur von ca. 40 °C gebracht. Anschließend wird eine wässrige Polyacrylsäure-Lösung zugegeben und weiter gerührt bis das Gemisch eine leicht trübe homogene Lösung ergibt. Die Lösung wird unter Rühren auf eine Temperatur von etwa 25 °C abgekühlt. Nach Zugabe von Aminomethylpropanol wird ein klares Gel gebildet.

### III. Polyurethanherstellung

### Herstellungsmethode A:

### Beispiel 3

In einem Rührapparat, der mit Rührer, Tropftrichter, Thermometer, Rückflusskühler und einer Vorrichtung für das Arbeiten unter Stickstoff ausgestattet war, wurden 600 g (0,3 mol) Oligomer O1, 600 g (0,4 mol) eines Polyesterdiols (Mₙ = 1500 g/mol, hergestellt aus Isophthalsäure, Adipinsäure, Neopentylglykol und Cyclohexandimethylol), 124,8 g (1,2 mol) Neopentylglykol, 375,2 g (2,8 mol) Dimethylolpropansäure und 0,5 g Tetrabutylorthotitanat in 760 g Methylethylketon unter Erhitzen auf eine Temperatur von etwa 80 °C und unter Rühren gelöst. Nachdem sich alles gelöst hatte, wurde das Reaktionsgemisch auf ca. 50 °C abgekühlt. Anschließend wurden unter Rühren 1111 g (5 mol) Isophorondiisocyanat zugetropft, wobei die Reaktionstemperatur anstieg. Unter Rückfluss wurde das Reaktionsgemisch dann solange gerührt, bis der Isocyanatgruppengehalt des Gemisches praktisch konstant blieb, und anschließend unter Rühren auf Raumtemperatur abgekühlt. Dann gab man zur Umsetzung der freien Isocyanatgruppen 276 g (3,1 mol) 2-Amino-2-methyl-1-propanol als wässrige Lösung bei einer Temperatur von etwa 40 °C zu dem Gemisch, um dieses zu 100 % zu neutralisieren. Die Flexibilitätseigenschaften der so erhaltenen Polymere sind in Tabelle 2 wiedergegeben. Nach Abdestillieren des Lösungsmittels im Vakuum bei 40 °C erhält man eine wässrige (Mikro)dispersion. Pulverförmige Produkte können durch Sprühtrocknen erhalten werden.

Analog dieser Herstellungsvorschrift wurden auch die Polymere V1, 2, 4-6 und 11 hergestellt.

Alle Carbonsäure-haltigen Polyurethane wurden mit 2-Amino-2-methyl-1-propanol zu 100 % neutralisiert. Die kationischen Polyurethane 11 und 12 wurden mit Milchsäure neutralisiert, Polyurethan 13 wurde mit Dimethylsulfat quaternisiert.

### Herstellungsvorschrift B:

### Beispiel 7

In einem Rührapparat, der mit Rührer, Tropftrichter, Thermometer, Rückflusskühler und einer Vorrichtung für das Arbeiten unter Stickstoff ausgestattet war, wurden 600 g (0,4 mol) eines Polyesterdiols (Mₙ = 1000 g/mol, hergestellt aus Isophthalsäure, Adipinsäure, Neopentylglykol und Cyclohexandimethylol), 187,2 g (1,8 mol) Neopentylglykol und 0,5 g Tetrabutylorthotitanat in 350 g Methylethylketon unter Erhitzen auf eine Temperatur von ca. 50 °C und unter Rühren gelöst. Anschließend wurden unter Rühren 252 g (1,5 mol) Hexamethylendiisocyanat zugetropft, wobei die Reaktionstemperatur anstieg. Unter Rückfluss wurde das Reaktionsgemisch etwa zwei Stunden gerührt und anschließend auf Raumtemperatur abgekühlt. Anschließend wurden 124,8 g (1,2 mol) Neopentylglykol, 375,2 g (2,8 mol) Dimethylolpropansäure und 200 g Methylethylketon zu dem Reaktionsgemisch gegeben und unter Erhitzen auf eine Temperatur von etwa 80 °C und unter Rühren gelöst. Nachdem sich alles gelöst hatte, wurde das Reaktionsgemisch auf ca. 50 °C abgekühlt. Anschließend wurde unter Rühren ein Gemisch aus 222 g (1 mol) Isophorondiisocyanat und 672 g (4 mol) Hexamethylendiisocyanat zugetropft, wobei die Reaktionstemperatur anstieg. Unter Rückfluss wurde das Reaktionsgemisch dann solange gerührt, bis der Isocyanatgruppengehalt praktisch konstant blieb und anschließend unter Rühren auf Raumtemperatur abgekühlt. Dann gab man zur Umsetzung der freien Isocyanatgruppen 276 g (3,1 mol) 2-Amino-2-methyl-1-propanol als wässrige Lösung bei einer Temperatur von etwa 40 °C zu dem Gemisch, um dieses zu 100 % zu neutralisieren. Nach Abdestillieren des Lösungsmittels im Vakuum bei 40 °C erhält man eine wässrige (Mikro)dispersion. Pulverförmige Produkte können durch Sprühtrocknen erhalten werden.

Analog dieser Herstellungsvorschrift wurden auch die Polymere 8-10, 12 und 13 hergestellt.

Die Beurteilung der anwendungstechnischen Eigenschaften erfolgte durch Notenvergabe für die Flexibilität von unabhängigen Fachleuten. Die Notenskala ist in Tabelle 6 wiedergegeben.

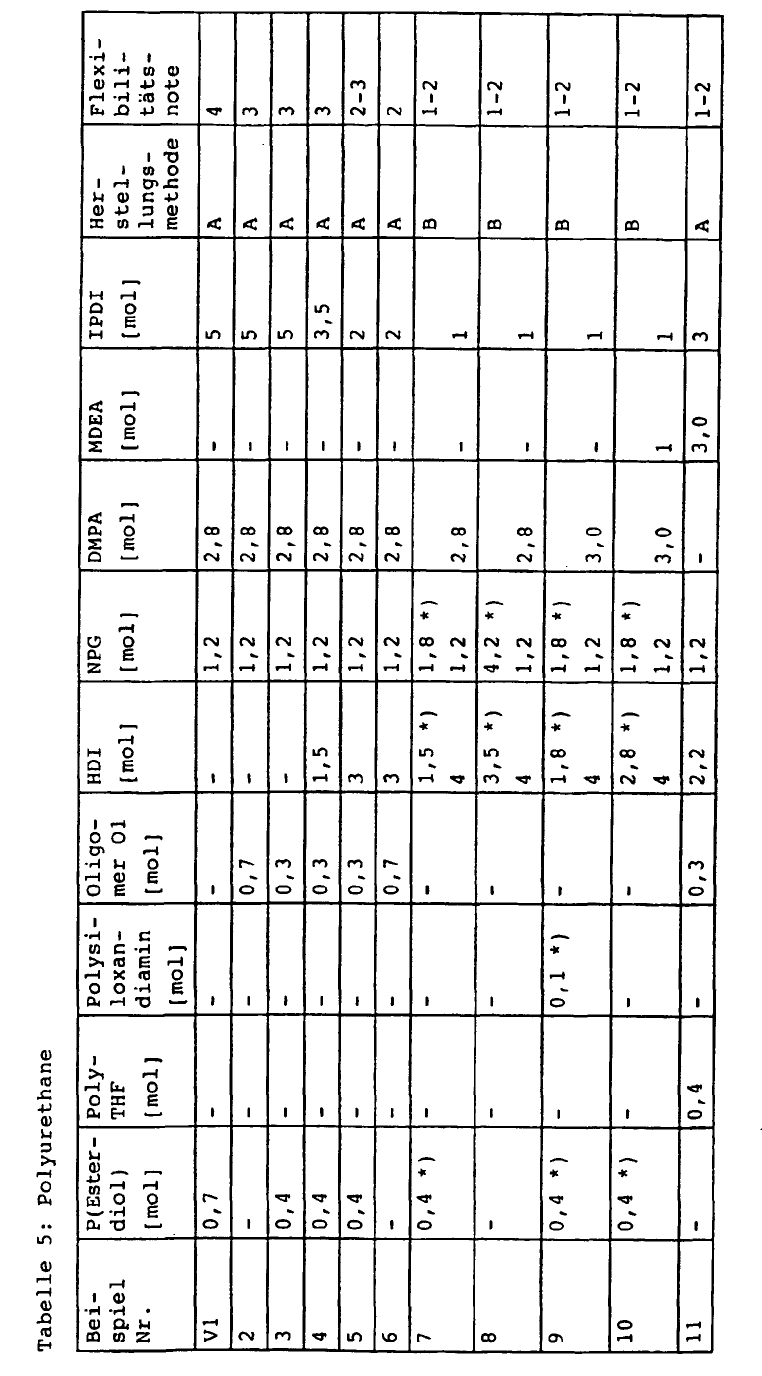

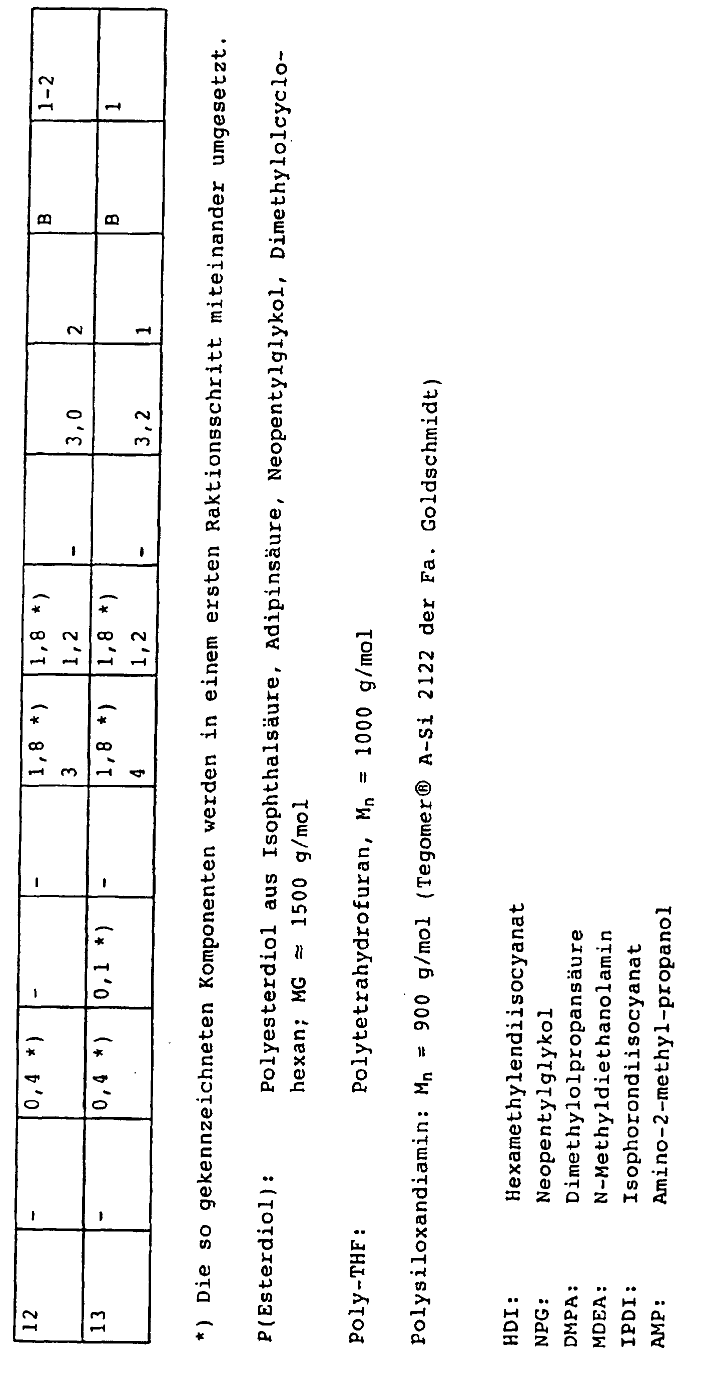

**Tabelle 6: Flexibilität**

| Note | Flexibilität |
|---|---|
| 1 | sehr flexibel |
| 2 | flexibel |
| 3 | mäßig flexibel |
| 4 | spröde |

Anwendungstechnische Beispiele

### Beispiele 14 bis 25

Aerosol-Haarspray-Formulierungen mit einem VOC-Gehalt von 97 Gew.-%:

| | |
|---|---|
| Polyurethan gemäß Beisp. 2-13 | 3,00 Gew.-% |
| Ethanol | 57,00 Gew.-%- |
| Dimethylether | 34,96 Gew.-% |
| Parfüm, Zusatzstoffe (u. a. Erucasäureester) | q.s. |

### Beispiele 26 bis 37

Kompakte Aerosol-Haarspray-Formulierungen mit einem VOC-Gehalt von 80 Gew.-%:

| | |
|---|---|
| Polyurethan gemäß Beisp. 2-13 | 5,00 Gew.-% |
| Ethanol | 40,00 Gew.-% |
| Dimethylether | 34,96 Gew.-% |
| Wasser | 15,00 Gew.-% |
| Parfüm, Zusatzstoffe | q.s. |

### Beispiele 38 bis 49

Haarspray-Formulierungen mit einem VOC-Gehalt von 55 Gew.-%:

| | |
|---|---|
| Polyurethan gemäß Beisp. 2-13 | 3,00 Gew.-% |
| Ethanol | 20,00 Gew.-% |
| Wasser | 42,00 Gew.-% |
| Dimethylether | 34,96 Gew.-% |
| Parfüm, Zusatzstoffe | q.s. |

### Beispiele 50 bis 61

Pump-Haarspray:

| | |
|---|---|
| Polyurethan gemäß Beisp. 2-13 | 5,00 Gew.-% |
| Ethanol | 54,96 Gew.-% |
| Wasser | 40,00 Gew.-% |
| Parfüm, Zusatzstoffe | q.s. |

### Beispiele 62 bis 73

**Schaum-Conditioner :[Gew.-%]**

| | |
|---|---|
| Polymer 2-13 (25%ige wässrige | |
| Lösung | 20,00 |
| Cremophor® A25⁷⁾ | 0,20 |
| Comperlan® KD⁸⁾ | 0,10 |
| Wasser | 69,70 |
| Propan/Butan | 9,96 |
| Parfüm, Konservierungsmittel | q.s. |

| | |
|---|---|
| ⁷⁾ CTFA-Name: Ceteareth 25, Fa. BASF AG, Umsetzungsprodukt aus Fettalkohol und Ethylenoxid | |
| ⁸⁾ CTFA-Name: Coamide DEA, Fa. Henkel, Kokosfettsäureamid | |

Zur Herstellung der Schaum-Conditioner werden die Komponenten eingewogen und unter Rühren gelöst. Anschließend werden sie in einen Spender abgefüllt und das Treibgas zugesetzt.

### Beispiele 74 bis 85

Aerosol-Haarspray mit einem VOC-Gehalt von 80 Gew.-% auf Basis von Polyurethanoligomeren

| | |
|---|---|
| Polymer gemäß Beisp. 2-13 | 5,00 Gew.-% |
| Oligomer 2-8 | 0,10 Gew.-% |
| Ethanol | 40,00 Gew.-% |
| Dimethylether | 34,96 Gew.-% |
| Wasser | 14,90 Gew.-% |
| Parfüm, Zusatzstoffe | q.s. |

### Beispiele 86 bis 88

| Conditioner-Shampoo: | | [Gew.-%] |
|---|---|---|
| A) | Texapon® NSO 28%ig⁹⁾ | 50,00 |
| | Comperlan® KD | 1,00 |
| | Polymer 11-13 (25%ige wässrige | |
| | Lösung | 20,00 |
| | Parfümöl | q.s. |
| B) | Wasser | 27,5 |
| | Natriumchlorid | 1,5 |
| | Konservierungsmittel | q.s. |

| | | |
|---|---|---|
| ⁹⁾ Natriumlaurylsulfat, Fa. Henkel | | |

Zur Herstellung der Conditioner-Shampoos werden die Komponenten A) und B) getrennt eingewogen und unter Mischen gelöst. Dann wird Phase B) langsam unter Rühren zu Phase A) gegeben.

### Beispiele für Anwendungen in der Hautkosmetik

### Beispiele 89 bis 95

### O/W-Cremes

| Ölphase: | Gew.-% | CTFA-Name: |
|---|---|---|
| Oligomer 2-8 | 1,1 | |
| Cremophor® A6 (BASF AG) | 3,5 | Ceteareth-6 (Stearylalkohol-Ethoxylat) |
| Cremophor® A25 (BASF AG) | 3,5 | Ceteareth-25 (Fettalkohol-Ethoxylat) |
| Glycerinmonostearat s.e. | 2,5 | Glycerylstearate |
| Paraffinöl | 7,5 | |
| Cetylalkohol | 3,5 | |
| Luvitol® EHO (BASF AG) | 3,2 | Cetearyloctanoat |
| Nip-Nip®, Nipa Laboratories Ltd., USA | 0,1 | Methyl- und Propyl-4-hydroxybenzoate (7:3) |

| Wasserphase: | Gew.-% | |
|---|---|---|
| Wasser | 75,0 | |
| Germall II, Sutton Laboratories Inc., USA | 0,1 | Diazolidinylharnstoff |

zur Herstellung der Cremes werden die Komponenten für Öl- und wasserphase getrennt eingewogen und bei 80 °C homogenisiert. Dann gibt man die Wasserphase langsam unter Rühren zu der Ölphase. Anschließend lässt man unter Rühren auf Raumtemperatur abkühlen.

### Beispiele 96 bis 107

### O/W-Cremes

| Ölphase: | Gew.-% | CTFA-Name: |
|---|---|---|
| Cremophor® A6 (BASF AG) | 3,5 | Ceteareth-6 (Stearylalkohol-Ethoxylat) |
| Cremophor® A25 (BASF AG) | 3,5 | Ceteareth-25 (Fettalkohol-Ethoxylat) |
| Glycerinmonostearat s.e. | 2,5 | Glycerylstearate |
| Paraffinöl | 7,5 | |
| Cetylalkohol | 3,5 | |
| Luvitol® EHO (BASF AG) | 3,2 | Cetearyloctanoat |
| Nip-Nip®, Nipa Laboratories Ltd., USA | 0,1 | Methyl- und Propyl-4-hydroxybenzoate (7:3) |

| Wasserphase: | Gew.-% | |
|---|---|---|
| Polymer 2-13 | 1,5 | |
| Wasser | 74,6 | |
| Germall II, Sutton Laboratories Inc., USA | 0,1 | Diazolidinylharnstoff |

Zur Herstellung der Cremes werden die Komponenten für Öl- und Wasserphase getrennt eingewogen und bei 80 °C homogenisiert. Dann gibt man die Wasserphase langsam unter Rühren zu der Ölphase. Anschließend lässt man unter Rühren auf Raumtemperatur abkühlen.

## Patentansprüche

1. Kosmetisches Mittel, enthaltend:
wenigstens ein Oligomer aus
A) mindestens einem aliphatischen Diisocyanat,
B) mindestens einer Verbindung mit wenigstens zwei gegenüber Isocyanatgruppen reaktiven Gruppen, die ausgewählt ist unter
B1) aliphatischen und cycloaliphatischen Polyolen, Polyaminen und/oder Aminoalkoholen,
B2) Polyetherolen und/oder Diaminopolyethern,
B3) Polysiloxanen mit wenigstens zwei aktiven Wasserstoffatomen pro Molekül,
B4) Polyesterpolyolen,
und Mischungen davon, und
c) gegebenenfalls mindestens einer Dicarbonsäure und/oder Hydroxycarbonsäure,
wobei das Oligomer pro Molekül wenigstens zwei Urethan- und/oder Harnstoffgruppen und zusätzlich wenigstens zwei weitere funktionelle Gruppen, die ausgewählt sind unter Hydroxyl-; primären und/oder sekundären Aminogruppen, umfasst und wobei Oligomere aus den Komponenten A) und B) ein Molekulargewicht im Bereich von 500 bis 7000 und Oligomere aus den Komponenten A), B) und C) ein Molekulargewicht im Bereich von 500 bis 10000 aufweisen.

2. Mittel nach Anspruch 1, enthaltend wenigstens ein Oligomer, welches mindestens eine Komponenten B4) eingebaut enthält, die ausgewählt ist unter Estern zwei- oder mehrwertiger Alkohole mit mindestens einer Carbonsäure, wobei die Carbonsäure ausgewählt ist unter
- cyclischen und acyclischen Dicarbonsäuren, erhalten durch Dimerisierung ungesättigter C₆- bis C₃₀-Carbonsäuren,
- aliphatischen, cycloaliphatischen und aromatischen C₈₋bis C₃₀-Dicarbonsäuren,
- C₈- bis C₃₀-Hydroxycarbonsäuren
und Mischungen davon.

3. Mittel nach Anspruch 1, enthaltend wenigstens ein Oligomer aus den Komponenten A), B1) und C).

4. Mittel nach Anspruch 3, wobei das Oligomer erhältlich ist durch Umsetzung mindestens einer Verbindung aus den Komponenten A) und B1) der allgemeinen Formel I
HO-R¹-A¹-CO-NH-R²-NH-CO-A¹-R¹-OH (I)
worin
R¹ für C₂- bis C₁₂-Alkylen, C₅- bis C₈-Cycloalkylen oder Arylen steht, wobei Alkylenreste durch einen oder zwei C₅₋bis C₈-Cycloalkylen- oder Arylenreste unterbrochen sein können,
R² für einen von einem aliphatischen, cycloaliphatischen oder aromatischen Diisocyanat nach Entfernen der Isocyanatgruppen abgeleiteten Rest steht,
A¹ für O oder NR³ steht, wobei R³ für Wasserstoff, C₁- bis C₆-Alkyl oder C₅- bis C₈-Cycloalkyl steht,
mit mindestens einer Verbindung der Komponente C), die ausgewählt ist unter cyclischen oder acyclischen Dicarbonsäuren, erhalten durch Dimerisierung ungesättigter C₆- bis C₃₀-Carbonsäuren, aliphatischen, cycloaliphatischen und aromatischen C₈- bis C₃₀-Dicarbonsäuren, C₈- bis C₃₀-Bydroxycarbonsäuren, und Mischungen davon.

5. Verwendung von Oligomeren, wie in einem der Ansprüche 1 bis 4 definiert, als Komponente pharmazeutischer und kosmetischer Zubereitungen, bevorzugt in kosmetischen Zubereitungen zur Behandlung der Haut oder der Haare, zur Modifizierung der rheologischen Eigenschaften von Zusammensetzungen auf Basis von Verbindungen geringer. Polarität sowie als Zwischenprodukte zur Herstellung wasserlöslicher oder wasserdispergierbarer Polyurethane.

6. Verwendung von Oligomeren, wie in einem der Ansprüche 1 bis 4 definiert, als oder in Beschichtungsmittel (n) und als oder in Behandlungsmittel(n) für nichtsaugfähige Oberflächen, bevorzugt Metalle, Kunststoffe, Textilsynthesefasern und Glas, und für saugfähige Oberflächen, bevorzugt Holz, Papier, Baumwolle und Leder, sowie als Verdickungsmittel für Flüssigkeiten niederer Polarität.

7. Oligomere ans den komponenten A) und B4) and aus den komponenten A), B1) und C), wie in einem der Ansprüche 1 bis 4 definiert.

8. Wasserlösliche oder wasserdispergierbare Polyurethane aus :
a) mindestens einem Oligomer, wie in einem der Ansprüche 1 bis 4 definiert,
b) mindestens einer Verbindung mit einem Molekulargewicht im Bereich von 56 bis 600, die zwei aktive Wasserstoffatome pro Molekül enthält,
c) mindestens einer Verbindung, die zwei aktive wasserstoffatome und mindestens eine ionogene und/oder ionische Gruppe pro Molekül -aufweist,
d) gegebenenfalls mindestens einem Polymerisat mit mindestens zwei aktiven Wasserstoffatomen pro Molekül,
e) mindestens einem Diisocyanat.

9. verfahren zur Herstellung eines Polyurethans, wie in Anspruch 8 definiert, durch Umsetzung wenigstens eines Oligomers a) und der Verbindungen der Komponenten b), c) und gegebenenfalls d) mit der Diisocyanatkomponente e), wobei die Herstellung der oligomere a) und die Herstellung den Polyuerethans ohne Isolierung eines Zwischenproduktes erfolgt.

10. Kosmetisches oder pharmazeutisches Mittel, das wenigstens ein Polyurethan nach Anspruch 8 enthält.

11. Kosmetisches Mittel nach Anspruch 10 in Form eines Haarbehandlungsmittels, enthaltend
a) 0,5 bis 20 Gew.-% mindestens eines Polyurethane, wie in Anspruch 8 definiert,
b) 50 bis 99,5 Gew.-% wenigstens eines Lösungemittels, ausgewähle unter Wasser; wassermischbaren Lösungsmitteln und Mischungen davon,
c) 0 bis 70 Gew.-%, bevorzugt 0,1 bis 50 Gew.-%; eines Treibmittels,
d) 0 bis 10 Gew.-% eines von a) verschiedenen, in wasser löslichen oder dispergierbaren Baarpolymers,
e) 0 bis 0,5 Gew.-% einer wasserlöslichen oder wasserdispergierbaren Siliconverbindung,
f) 0 bis 1 Gew.-%, bevorzugt 0,0001 bis 0,5 Gew.-%, wenigstens einer Verbindung, die ausgewählt ist unter Estern und Amiden von gesättigten und einfach oder mehrfach ungesättigten C₅- bis C₃₀-Carbonsäuren, gesättigten und einfach oder mehrfach ungesättigten C₈- bis C₃₀-Alkoholen und Mischungen davon.

12. Verwendung der Polyurethane, wie in Anspruch 8 definiert, als Hilfsmittel in der Kosmetik, bevorzugt als Beschichtungsmittel für Haar, Baut und Nägel, insbesondere in der Haarkosmetik, vorzugsweise als Festigerpolymer in Haarsprays, Schaumfestigern, Haarmousse, Haargel und Shampoos.

13. Verwendung der Polyurethane, wie in Anspruche 8 definiert, in der dekorativen Kosmetik, bevorzugt in Mascara, Make-up und Lidschatten, als polymerer Emulgator oder Coemulgator zur Formulierung von kosmetischen oder pharmazeutischen Präparaten sowie als Hilfsmittel in der Pharmazie, bevorzugt als oder in Beschichbungsmittel(n) oder Bindemittel(n) für feste Arzneiformen.

14. Verwendung der Polyurethane, wie in Anspruch 8 definiert, als oder in Beschichtungsmittel(n) für die Textil-, Papier-, Druck-, Leder- und Klebstoffindustrie sowie als Polymeremulgator für nicht-kosmetische Zubereitungen.

## Claims

1. A cosmetic composition comprising:
at least one oligomer of
A) at least one aliphatic diisocyanate,
B) at least one compound having at least two groups which are reactive toward isocyanate groups, which is chosen from
B1) aliphatic and cycloaliphatic polyols, polyamines and/or aminoalcohols,
B2) polyetherols and/or diaminopolyethers,
B3) polysiloxanes having at least two active hydrogen atoms per molecule,
B4) polyester polyols,
and mixtures thereof, and
C) optionally at least one dicarboxylic acid and/or hydroxycarboxylic acid,
where the oligomer comprises, per molecule, at least two urethane and/or urea groups and additionally at least two further functional groups chosen from hydroxyl, primary and/or secondary amino groups, and where oligomers from components A) and B) have a molecular weight in the range from 500 to 7 000 and oligomers of components A), B) and C) have a molecular weight in the range from 500 to 10 000.

2. The composition according to claim 1, comprising at least one oligomer which comprises, in incorporated form, at least one component B4) chosen from esters of di- or polyhydric alcohols with at least one carboxylic acid, the carboxylic acid being chosen from
- cyclic and acyclic dicarboxylic acids, obtained by dimerization of unsaturated C₆- to C₃₀-carboxylic acids,
- aliphatic, cycloaliphatic and aromatic C₈- to C₃₀-dicarboxylic acids,
- C₈- to C₃₀-hydroxycarboxylic acids
and mixtures thereof.

3. The composition according to claim 1, comprising at least one oligomer from the components A), B1) and C).

4. The composition according to claim 3, where the oligomer is obtainable by reacting at least one compound from the components A) and B1) of the formula I
HO-R¹-A¹-CO-NH-R²-NH-CO-A¹-R¹-OH (I)
in which
R¹ is C₂- to C₁₂-alkylene, C₅- to C₈-cycloalkylene or arylene, where alkylene radicals may be interrupted by one or two C₅- to C₈-cycloalkylene or arylene radicals,
R² is a radical derived from an aliphatic, cycloaliphatic or aromatic diisocyanate following removal of the isocyanate groups,
A¹ is O or NR³, where R³ is hydrogen, C₁- to C₆-alkyl or C₅- to C₈-cycloalkyl,
with at least one compound of component C), which is chosen from cyclic or acyclic dicarboxylic acids, obtained by dimerization of unsaturated C₆- to C₃₀-carboxylic acids, aliphatic, cycloaliphatic and aromatic C₈- to C₃₀-dicarboxylic acids, C₈- to C₃₀-hydroxycarboxylic acids, and mixtures thereof.

5. The use of oligomers as defined in any of claims 1 to 4 as a component of pharmaceutical and cosmetic preparations, preferably in cosmetic preparations for the treatment of the skin or of the hair, for the modification of rheological properties of compositions based on compounds of low polarity, and as intermediates for the preparation of water-soluble or water-dispersible polyurethanes.

6. The use of oligomers as defined in any of claims 1 to 4 as or in coating(s) and as or in treatment agent(s) for nonabsorbent surfaces, preferably metals, plastics, synthetic textile fibers and glass, and for absorbent surfaces, preferably wood, paper, cotton and leather, and as thickeners for liquids of low polarity.

7. An oligomer of components A) and B4) and of components A), B1) and C), as defined in any of claims 1 to 4.

8. A water-soluble or water-dispersible polyurethane of:
a) at least one oligomer, as defined in any of claims 1 to 4,
b) at least one compound having a molecular weight in the range from 56 to 600, which comprises two active hydrogen atoms per molecule,
c) at least one compound which has two active hydrogen atoms and at least one ionogenic and/or ionic group per molecule,
d) if appropriate at least one polymer having at least two active hydrogen atoms per molecule,
e) at least one diisocyanate.

9. A process for the preparation of a polyurethane, as defined in claim 8, by reacting at least one oligomer a) and the compounds of components b), c) and if appropriate d) with the diisocyanate component e), where the preparation of the oligomers a) and the preparation of the polyurethane takes place without isolation of an intermediate.

10. A cosmetic or pharmaceutical composition which comprises at least one polyurethane according to claim 8.

11. The cosmetic composition according to claim 10, in the form of a hair-treatment composition, comprising
a) 0.5 to 20% by weight of at least one polyurethane, as defined in claim 8,
b) 50 to 99.5% by weight of at least one solvent, chosen from water, water-miscible solvents and mixtures thereof,
c) 0 to 70% by weight, preferably 0.1 to 50% by weight, of a propellant,
d) 0 to 10% by weight of a water-soluble or -dispersible hair polymer which is different from a),
e) 0 to 0.5% by weight of a water-soluble or water-dispersible silicone compound,
f) 0 to 1% by weight, preferably 0.0001 to 0.5% by weight, of at least one compound chosen from esters and amides of saturated and mono- or polyunsaturated C₅- to C₃₀-carboxylic acids, saturated and mono- or polyunsaturated C₈- to C₃₀-alcohols and mixtures thereof.

12. The use of the polyurethanes as defined in claim 8 as auxiliaries in cosmetics, preferably as coatings for hair, skin and nails, in particular in hair cosmetics, preferably as setting polymers in hair sprays, setting foams, hair mousse, hair gel and shampoos.

13. The use of the polyurethanes as defined in claim 8 in decorative cosmetics, preferably in mascara, make-up and eyeshadows, as polymeric emulsifier or co-emulsifier for the formulation of cosmetic or pharmaceutical preparations, and as auxiliaries in pharmacy, preferably as or in coating(s) or binder(s) for solid medicament forms.

14. The use of the polyurethanes as defined in claim 8 as or in coating(s) for the textile, paper, printing, leather and adhesives industry, and as polymer emulsifier for non-cosmetic preparations.

## Revendications

1. Produit cosmétique, contenant :
au moins un oligomère à base
A) d'au moins un diisocyanate aliphatique,
B) d'au moins un composé qui comporte au moins deux groupes réactifs vis-à-vis des groupes isocyanate et qui est choisi parmi
B1) des aminoalcools, des polyamines et/ou des polyols aliphatiques et cycloaliphatiques,
B2) des polyétherols et/ou des diaminopolyéthers,
B3) des polysiloxanes comportant au moins deux atomes d'hydrogène actifs par molécule,
B4) des polyesterpolyols,
et leurs mélanges, et
C) éventuellement d'au moins un acide dicarboxylique et/ou acide hydroxycarboxylique,
l'oligomère comportant par molécule au moins deux groupes uréthanne et/ou urée et en supplément au moins deux autres groupes fonctionnels qui sont choisis parmi des groupes hydroxyle, amino primaires et/ou secondaires, les oligomères à base des composants A) et B) présentant un poids moléculaire de l'ordre de 500 à 7000 et les oligomères à base des composants A), B) et C) un poids moléculaire de l'ordre de 500 à 10.000.

2. Produit suivant la revendication 1, contenant au moins un oligomère qui contient à l'état incorporé au moins un composant B4) qui est choisi parmi des esters d'alcools bifonctionnels ou polyfonctionnels avec au moins un acide carboxylique, l'acide carboxylique étant choisi parmi
- des acides dicarboxyliques cycliques et acycliques, obtenus par dimérisation d'acides carboxyliques en C₆-C₃₀ insaturés,
- des acides dicarboxyliques en C₈-C₃₀ aliphatiques, cycloaliphatiques et aromatiques,
- des acides hydroxycarboxyliques en C₈-C₃₀, et leurs mélanges.

3. Produit suivant la revendication 1, contenant au moins un oligomère à base des composants A), B1) et C) .

4. Produit suivant la revendication 3, dans lequel l'oligomère peut être obtenu par réaction d'au moins un composé à base des composants A) et B1) de la formule générale I :
HO-R¹-A¹-CO-NH-R²-NH-CO-A¹-R¹-OH (I)
dans laquelle
R¹ représente un groupe alkylène en C₂-C₁₂, cycloalkylène en C₅-C₈ ou arylène, les radicaux alkylène pouvant être interrompus par un ou deux radicaux cycloalkylène en C₅-C₈ ou arylène,
R² représente un diisocyanate aliphatique, cycloaliphatique ou aromatique après élimination du radical dérivé des groupes isocyanate,
A¹ représente O ou NR³, R³ représentant de l'hydrogène ou un groupe alkyle en C₁-C₆ ou cycloalkyle en C₅-C₈,
avec au moins un composé du composant C), qui est choisi parmi des acides dicarboxyliques cycliques ou acycliques, obtenus par dimérisation d'acides carboxyliques en C₆-C₃₀ insaturés, des acides dicarboxyliques en C₈-C₃₀ aliphatiques, cycloaliphatiques et aromatiques, des acides hydroxycarboxyliques en C₈-C₃₀ et leurs mélanges.

5. Utilisation d'oligomères tels que définis dans l'une des revendications 1 à 4, comme composants de compositions pharmaceutiques et cosmétiques, avantageusement dans des compositions cosmétiques destinées au traitement de la peau ou des cheveux, à la modification des propriétés rhéologiques de compositions à base de composés de faible polarité, ainsi que comme produits intermédiaires pour la préparation de polyuréthannes solubles dans l'eau ou dispersables dans l'eau.

6. Utilisation d'oligomères tels que définis dans l'une des revendications 1 à 4, comme ou dans un ou des produits d'enduction et comme ou dans un ou des produits de traitement de surfaces non absorbantes, de préférence des métaux, des substances synthétiques, des fibres de synthèse textiles et du verre, et pour des surfaces absorbantes, de préférence du bois, du papier, du coton et du cuir, ainsi que comme agents d'épaississement de liquides de polarité basse.

7. Oligomères à base des composants A) et B4) et à base des composants A), B1) et C), comme défini dans l'une des revendications 1 à 4.

8. Polyuréthannes solubles ou dispersables dans l'eau, à base :
a) d'au moins un oligomère, comme défini dans l'une des revendications 1 à 4,
b) d'au moins un composé présentant un poids moléculaire de l'ordre de 56 à 600, qui contient deux atomes d'hydrogène actifs par molécule,
c) d'au moins un composé qui présente deux atomes d'hydrogène actifs et au moins un groupe ionogène et/ou ionique par molécule,
d) éventuellement d'au moins un polymère comportant au moins deux atomes d'hydrogène actifs par molécule,
e) d'au moins un diisocyanate.

9. Procédé de préparation d'un polyuréthanne tel que défini dans la revendication 8, par réaction d'au moins un oligomère a) et des composés des composants b), c) et éventuellement d) avec le composant de diisocyanate e), la préparation des oligomères a) et la préparation du polyuréthanne ayant lieu sans isolement d'un produit intermédiaire.

10. Produit cosmétique ou pharmaceutique, qui contient au moins un polyuréthanne selon la revendication 8.

11. Produit cosmétique suivant la revendication 10, sous la forme d'un produit de traitement des cheveux, contenant
a) 0,5 à 20 % en poids d'au moins un polyuréthanne, tel que défini dans la revendication 8,
b) 50 à 99,5 % en poids d'au moins un solvant, choisi parmi de l'eau, des solvants miscibles à l'eau et leurs mélanges,
c) 0 à 70 % en poids, de préférence 0,1 à 50 % en poids, d'un agent moussant,
d) 0 à 10 % en poids d'un polymère pour les cheveux soluble ou dispersable dans l'eau, différent de a),
e) 0 à 0,5 % en poids d'un composé de silicone soluble ou dispersable dans l'eau,
f) 0 à 1 % en poids, de préférence 0,0001 à 0,5 % en poids, d'au moins un composé qui est choisi parmi des esters et des amides d'acides carboxyliques en C₅-C₃₀ saturés et insaturés à une ou plusieurs reprises et des alcools en C₈-C₃₀ saturés et insaturés à une ou plusieurs reprises, et leurs mélanges.

12. Utilisation des polyuréthannes tels que définis dans la revendication 8, comme adjuvant dans la cosmétique, de préférence comme produit d'enduction pour des cheveux, la peau et les ongles, en particulier dans la cosmétique des cheveux, de préférence comme polymère fixateur dans des pulvérisations pour cheveux, renforçateurs de mousse, mousses capillaires, gels capillaires et shampooings.

13. Utilisation des polyuréthannes tels que définis dans la revendication 8, dans la cosmétique décorative, de préférence dans du mascara, du maquillage et du fard à paupières, comme agent émulsionnant ou coémulsionnant polymère destiné à la formulation de préparations cosmétiques ou pharmaceutiques, ainsi que comme adjuvant en pharmacie, de préférence comme ou dans un ou des produits d'enduction ou un ou des liants pour des formes médicamenteuses solides.

14. Utilisation des polyuréthannes tels que définis dans la revendication 8, comme ou dans un ou des produits d'enduction pour l'industrie des matières textiles, du papier, de l'impression, du cuir et des vêtements ainsi que comme agent émulsionnant polymère pour des compositions non cosmétiques.
